(19) Europäisches Patentamt
European Patent Office
Office européen des brevets

(11) Veröffentlichungsnummer: **0 071 175**
**B1**

(12) **EUROPÄISCHE PATENTSCHRIFT**

(45) Veröffentlichungstag der Patentschrift: **12.03.86**

(51) Int. Cl.⁴: **C 07 D 303/48,** A 61 K 31/335, C 07 C 69/612, C 07 C 67/343

(21) Anmeldenummer: **82106573.7**

(22) Anmeldetag: **21.07.82**

(54) **Phenylalkyloxirancarbonsäuren, Verfahren zu ihrer Herstellung, ihre Anwendung und sie enthaltende Arzneimittel.**

(30) Priorität: **24.07.81 CH 4838/81**

(43) Veröffentlichungstag der Anmeldung: **09.02.83 Patentblatt 83/6**

(45) Bekanntmachung des Hinweises auf die Patenterteilung: **12.03.86 Patentblatt 86/11**

(84) Benannte Vertragsstaaten: **AT BE CH DE FR GB IT LI LU NL SE**

(56) Entgegenhaltungen:
**EP - A - 0 025 192**

**I. MED. CHEM. 1982, 25, 109-113**

**Die Akte enthält technische Angaben, die nach dem Eingang der Anmeldung eingereicht wurden und die nicht in dieser Patentschrift enthalten sind.**

(73) Patentinhaber: **Byk Gulden Lomberg Chemische Fabrik GmbH, Byk-Gulden-Strasse 2, D-7750 Konstanz (DE)**

(72) Erfinder: **Kohl, Bernhard, Dr., Heinrich-v.-Tettingenstrasse 35a, D-7750 Konstanz 21 (DE)**
Erfinder: **Eistetter, Klaus, Dr., Säntisblick 7, D-7750 Konstanz 19 (DE)**
Erfinder: **Amschler, Hermann, Dr., Hohenhewenstrasse 19, D-7760 Radolfzell (DE)**
Erfinder: **Ludwig, Gerhard, Dr., Meisenweg 6, D-7750 Konstanz 16 (DE)**
Erfinder: **Wolf, Horst, Dr., Brandesstrasse 29, D-7750 Konstanz (DE)**

**EP 0 071 175 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Herstellung betrifft Phenylalkyloxirancarbonsäuren, Verfahren zu ihrer Herstellung, ihre Anwendung und sie enthaltende Arzneimittel.

Im Rahmen einer Untersuchung über die Fähigkeit von substituierten Dreiringverbindungen, als Substrat oder Inhibitor für Meerschweinchen-Lebermicrosomen-Epoxihydrase zu dienen, wurden u.a. Phenyloxirancarbonsäureester, z.B. 2-Phenyl-oxiran -2- carbonsäureethylester, eingesetzt [F. Ösch et al., Biochem. 10(1971) No. 26, 4858–66]. Im Verlauf der Strukturaufklärung von Isamsäure [P. de Mayo und J.J. Ryan, Can. J. Chem. 45(1967) 2177–2190] wurde Methyl -2-(o-acetaminobenzyl)-glycidat durch Reaktion von Methyl-N-acetylisatinat mit überschüssigem Diazomethan erhalten. Substituierte Oxirancarbonsäuren werden in der deutschen Offenlegungsschrift DE-OS 30 32 669 beschrieben. Über die Kondensation von Benzaldehyden mit Ethylidenmalonester bei Herstellung von Cannabinoiden wird von L. Crombie et al. (Tetrahedron Letters 1979, 49, 4773–76) berichtet.

Gegenstand der Erfindung sind Phenylalkyloxirancarbonsäuren der allgemeinen Formel I

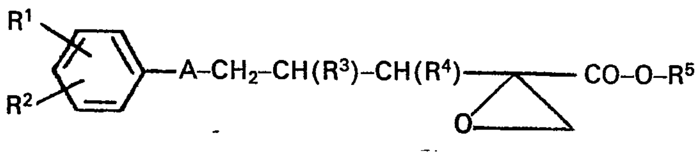

(I),

worin

$R^1$ ein Wasserstoffatom, ein Halogenatom, eine C1–C4-Niederalkylgruppe, eine C1–C4-Niederalkoxygruppe oder eine Trifluormethylgruppe bedeutet,

$R^2$ eine der Bedeutungen von $R^1$ hat,

A eine Einfachbindung, eine $-CH(R^6)-CH(R^7)$-Gruppe, eine $-CH(R^6)-CH(R^7)-CH(R^8)$-Gruppe oder eine $-CH(R^6)-CH(R^7)-CH(R^8)-CH(R^9)$-Gruppe bedeutet,

$R^5$ ein Wasserstoffatom oder eine C1–C4-Niederalkylgruppe bedeutet, einer der Substituenten $R^3$, $R^4$, $R^6$, $R^7$, $R^8$ oder $R^9$ eine C1–C4-Niederalkylgruppe darstellt und die anderen Wasserstoffatome bedeuten, und die Salze der Carbonsäuren.

Als C1–C4-Niederalkylgruppen kommen geradkettige oder verzweigte Alkylreste mit 1 bis 4 Kohlenstoffatomen in Betracht. Geradkettige Alkylreste sind beispielsweise der Methyl-, Ethyl-, n-Propyl- und n-Butylrest, von denen die mit 1 und 2 Kohlenstoffatomen bevorzugt sind. Verzweigte Alkylreste sind beispielsweise der Isopropyl-, Isobutyl- und sek.-Butylrest, von denen der mit 3 Kohlenstoffatomen bevorzugt ist. Als Alkylreste von C1–C4-Niederalkoxygruppen kommen sowohl geradkettige als auch verzweigte C1–C4-Niederalkylgruppen in Frage. Die Methoxygruppe ist als Niederalkoxygruppe bevorzugt.

Halogenatome sind Fluor-, Chlor- und Bromatome, von denen Fluor, insbesondere Chlor bevorzugt sind.

Die Substituenten $R^1$ und $R^2$ befinden sich bevorzugt in meta- oder para-Position zum Alkylenoxirancarbonsäurerest.

Als Salze kommen Salze mit anorganischen und organischen Basen in Betracht. Pharmakologisch nicht verträgliche Salze werden nach an sich bekannten Methoden in pharmakologisch, d.h. biologisch, verträgliche Salze übergeführt, die unter den erfindungsgemässen Salzen bevorzugt sind. Als Kationen für die Salzbildung werden vor allem die Kationen der Alkalimetalle, Erdalkalimetalle oder Erdmetalle verwendet, es kommen jedoch auch die entsprechenden Kationen organischer Stickstoffbasen, wie Amine, Aminoalkanole, Aminozucker, basischer Aminosäuren, etc. zur Anwendung.

Beispielsweise seien die Salze von Lithium, Natrium, Kalium, Magnesium, Calcium, Aluminium, Ethylendiamin, Dimethylamin, Diethylamin, Morpholin, Piperidin, Piperazin, N-Niedrigalkylpiperazinen (z.B. N-Methylpiperazin), Methylcyclohexylamin, Benzylamin, Ethanolamin, Diethanolamin, Triethanolamin, Tris-(hydroxymethyl)-aminomethan, 2-Amino -2- methylpropanol, 2-Amino -2- methyl-1,3-propandiol, Glucamin, N-Methylglucamin, Glucosamin, N-Methylglucosamin, Lysin, Ornithin, Arginin, Chinolin genannt.

Eine Ausgestaltung der Erfindung sind Phenylalkyloxirancarbonsäuren der allgemeinen Formel I, worin $R^1$ und $R^2$ meta- oder para-Substituenten sind und $R^1$ ein Wasserstoffatom, ein Chloratom, eine Methylgruppe oder eine Trifluormethylgruppe, $R^2$ ein Wasserstoffatom oder ein Chloratom, A eine Einfachbindung, eine $-CH(R^6)-CH(R^7)$-Gruppe oder eine $-CH(R^6)-CH(R^7)-CH(R^8)$-Gruppe bedeutet, $R^5$ ein Wasserstoffatom oder eine C1–C4-Niederalkylgruppe, einer der Substituenten $R^3$, $R^4$, $R^6$, $R^7$ oder $R^8$ eine Methyl- oder Ethylgruppe darstellt und die anderen Wasserstoffatome bedeuten, und die Salze der Carbonsäure.

Eine weitere Ausgestaltung der Erfindung sind Phenylalkyloxirancarbonsäuren der allgemeinen Formel I, worin $R^1$ und $R^2$ meta- oder para-Substituenten sind und $R^1$ ein Wasserstoffatom, ein Chloratom oder eine Trifluormethylgruppe, $R^2$ ein Wasserstoffatom, A eine Einfachbindung oder eine $-CH(R^6)-CH(R^7)$-Gruppe bedeutet, $R^5$ ein Wasserstoffatom, eine Methylgruppe oder eine Ethylgruppe bedeutet, einer der Substituenten $R^3$, $R^4$, $R^6$ oder $R^7$ eine Methylgruppe darstellt und die anderen ein Wasserstoffatom bedeuten, und die pharmakologisch verträglichen Salze der Carbonsäuren.

Bevorzugte Verbindungen sind solche der For-

mel I, worin $R^1$ und $R^2$ meta- oder para-Substituenten sind, $R^1$ ein Wasserstoffatom, ein Chloratom oder eine Trifluormethylgruppe, $R^2$ ein Wasserstoffatom, A eine Einfachbindung, eine $-CH(R^6)-CH(R^7)-$ oder $-CH(R^6)-CH(R^7)-CH(R^8)-CH(R^9)$-Gruppe, $R^5$ ein Wasserstoffatom oder eine Alkylgruppe mit 1 bis 4 Kohlenstoffatomen bedeutet, einer der Substituenten $R^3$, $R^4$, $R^6$, $R^7$, $R^8$ oder $R^9$ eine Methyl- oder Ethylgruppe und die anderen Wasserstoffatome darstellen, und die Salze der Carbonsäuren.

Besonders bevorzugte Verbindungen sind solche der Formel I, worin $R^1$ ein para-Substituent ist und ein Wasserstoffatom- oder Chloratom darstellt, $R^2$ ein Wasserstoffatom, A eine $-CH(R^6)-CH(R^7)-$ oder $-CH(R^6)-CH(R^7)-CH(R^8)-CH(R^9)$-Gruppe, $R^5$ ein Wasserstoffatom oder eine Methyl- oder Ethylgruppe, $R^4$ ein Wasserstoffatom, $R^8$ ein Wasserstoffatom bedeutet, einer der Substituenten $R^3$, $R^6$, $R^7$ oder $R^9$ eine Methyl- oder Ethylgruppe und die anderen Wasserstoffatome darstellen, und die pharmakologisch verträglichen Salze der Carbonsäuren.

Als Vertreter der erfindungsgemässen Verbindungen seien beispielsweise
2-(2-Ethyl -3- phenylpropyl)-oxiran -2- carbonsäureethylester,
2-[3-(3-Chlorphenyl) -1- methylpropyl]-oxiran -2- carbonsäuremethylester,
2-[2-Methyl -3- (3-trifluormethylphenyl)-propyl]-oxiran -2- carbonsäureethylester,
2-[3-(4-Bromphenyl) -2- propyl-propyl)-oxiran -2- carbonsäureethylester,
2-[5-(4-Fluorphenyl) -1- methylpentyl]-oxiran -2- carbonsäureisopropylester,
2-[5-(3-Chlorphenyl) -2- methylpentyl]-oxiran -2- carbonsäureethylester,
2-[4-Ethyl -5- (4-methylphenyl)-pentyl]-oxiran -2- carbonsäureethylester,
2-[4-Methyl -5- (3-trifluormethylphenyl)-pentyl]-oxiran -2- carbonsäure-n-butylester,
2-(2-Butyl -5- phenylpentyl)-oxiran -2- carbonsäureethylester,
2-[2-Isobutyl -5- (4-methoxyphenyl)-pentyl]-oxiran -2- carbonsäure-isopropylester,
2-(2-Methyl -6- phenylhexyl)-oxiran -2- carbonsäureethylester,
2-[6-(3-Chlorphenyl) -2- methylhexyl]-oxiran -2- carbonsäureethylester,
2-[7-(3,4-Dichlorphenyl) -5- methylheptyl]-oxiran -2- carbonsäuremethylester,
2-[2-Ethyl -7- (3-trifluormethylphenyl)-heptyl]-oxiran -2- carbonsäure-n-propylester,
2-[7-(4-Chlorphenyl) -4- methylheptyl]-oxiran -2- carbonsäureethylester,
2-(2-Methyl -3- phenylpropyl)-oxiran -2- carbonsäure-n-propylester,
2-[2-Ethyl -3- (4-fluorphenyl)-propyl]-oxiran -2- carbonsäureethylester,
2-[3-(4-Bromphenyl) -1- methylpropyl]-oxiran -2- carbonsäureethylester,
2-[3-(2-Chlorphenyl) -1- methylpropyl]-oxiran -2- carbonsäureethylester,
2-[2-n-Butyl -3- (4-chlorphenyl)-propyl]-oxiran -2- carbonsäureethylester,
2-[5-(2-Chlorphenyl) -5- ethylpentyl]-oxiran -2- carbonsäureethylester,
2-[5-(3,4-Dichlorphenyl) -5- methylpentyl]-oxiran -2- carbonsäureethylester,
2-[5-(2,4-Dichlorphenyl) -4- methylpentyl]-oxiran -2- carbonsäurepropylester,
2-[5-(3,4-Dichlorphenyl) -4- methylpentyl]-oxiran -2- carbonsäure-n-butylester,
2-[5-(2,4-Dichlorphenyl) -4- methylpentyl]-oxiran -2- carbonsäure-sec-butylester.
2-[5-(4-Chlorphenyl) -2- propylpentyl]-oxiran -2- carbonsäuremethylester,
2-[5-(4-Fluorphenyl) -2- isopropylpentyl]-oxiran -2- carbonsäureethylester,
2-[5-(4-Chlor -2- methylphenyl) -5- ethylpentyl]-oxiran -2- carbonsäureethylester,
2-[5-(4-Methoxyphenyl) -4- methylpentyl]-oxiran -2- carbonsäure-n-propylester,
die entsprechenden Phenylalkyloxiran -2- carbonsäuren sowie deren Salze mit anorganischen und organischen Basen genannt.

Bevorzugte Vertreter sind
2-(4-Methyl -5- phenylpentyl)-oxiran -2- carbonsäureethylester
2-(2-Ethyl -5- phenylpentyl)-oxiran -2- carbonsäureethylester
2-[5-(4-Chlorphenyl) -4- methylpentyl]-oxiran -2- carbonsäureethylester
2-(4-Methyl -7- phenylheptyl)-oxiran -2- carbonsäureethylester
2-(5-Methyl -5- phenylpentyl)-oxiran -2- carbonsäureethylester
die entsprechenden Phenylalkyloxiran -2- carbonsäuren sowie deren pharmakologisch verträglichen Salze.

Die Phenylalkyloxirancarbonsäuren der allgemeinen Formel I besitzen zwei Chiralitätszentren. Die Erfindung schliesst daher sowohl die Diastereomeren, die Racemate und die Enantiomeren als auch deren Gemische ein.

Die erfindungsgemässen Verbindungen weisen wertvolle pharmakologische Eigenschaften auf, die sie gewerblich verwertbar machen. Sie senken den Blutglucosespiegel und den Blutspiegel der Ketonkörper, wobei sie sich in ihrer chemischen Struktur und ihrer Wirksamkeit grundlegend von pankreaswirksamen, betacytotropen Substanzen (z.B. Sulfonylharnstoffen) durch ihre extrapankreatische Wirkung unterscheiden und extrapankreatisch wirkenden Handelspräparaten überlegen erweisen. Ausserdem zeichnen sie sich durch ihre Langzeitwirkung aus.

Aufgrund ihrer vorteilhaften Wirksamkeit sind die erfindungsgemässen substituierten Oxirancarbonsäuren der allgemeinen Formel I, sowie die pharmakologisch verträglichen Salze zur human- und veterinärmedizinischen Behandlung und

Prophylaxe von Krankheiten, die auf Störungen des Glucose- und Fettstoffwechsels beruhen, geeignet. Beispielsweise werden behandelt prädiabetische Zustände zur Verhinderung der Manifestation des Diabetes, manifester Diabetes, z.B. Erwachsenendiabetes, labiler Diabetes von Jugendlichen sowie alle krankhaften Zustände, die mit einer pathologisch erhöhten Ketonkörperproduktion einhergehen.

Gegenstand der Erfindung sind daher auch die erfindungsgemässen Verbindungen zur Anwendung bei der Behandlung der angegebenen Krankheiten.

Ein weiterer Gegenstand der Erfindung sind Arzneimittel, die eine oder mehrere der Phenylalkyloxirancarbonsäuren der allgemeinen Formel I und/oder die pharmakologisch verträglichen Salze der Säuren mit anorganischen oder organischen Basen enthalten.

Ausgestaltungen der Arzneimittel sind solche, die die bevorzugten oder besonders bevorzugten Phenylalkyloxirancarbonsäuren und/oder die pharmakologisch verträglichen Salze der Säuren enthalten.

Gegenstand der Erfindung ist ausserdem die Verwendung der erfindungsgemässen Verbindungen zur Herstellung von Arzneimitteln zur Bekämpfung der angegebenen Krankheiten.

Die Arzneimittel werden nach an sich bekannten, dem Fachmann geläufigen Verfahren hergestellt. Als Arzneimittel werden die erfindungsgemässen pharmakologisch wirksamen Verbindungen (= Wirkstoffe) entweder als solche, oder vorzugsweise in Form von Tabletten, Dragées, Kapseln, Suppositorien, Emulsionen, Suspensionen oder Lösungen eingesetzt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral oder parenteral appliziert werden. Im allgemeinen hat es sich in der Humanmedizin als vorteilhaft erwiesen, den oder die Wirkstoffe bei oraler Gabe in einer Tagesdosis von etwa 0,1 bis etwa 30, vorzugsweise 0,3 bis 15, insbesondere 0,6 bis 3 mg/kg Körpergewicht, gegebenenfalls in Form mehrerer, vorzugsweise 1 bis 4 Einzelangaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Neben den erfindungsgemässen Phenylalkyloxirancarbonsäuren, in denen die Substituenten die oben angegebene Bedeutung haben, und/oder ihren Salzen können die pharmazeutischen Zubereitungen weiterhin einen oder mehrere pharmakologisch aktive Bestandteile anderer Arzneimittelgruppen, wie Antidiabetika (Sulfonamide, Sulfonylharnstoffe u.a.), z.B. Carbutamid, Tolbutamid, Chlorpropamid, Glibenclamid, Glibornurid, Glisoxepid, Gliquidon, Glymidin oder Hypolipidämika, wie Nikotinsäure sowie deren Derivate und Salze, enthalten.

Ein weiterer Gegenstand der Erfindung ist ein Verfahren zur Herstellung von Phenylalkyloxirancarbonsäuren der allgemeinen Formel I und den Salzen der Carbonsäuren, das dadurch gekennzeichnet ist, dass man Phenylalkyl -2- methylencarbonsäuren der allgemeinen Formel II

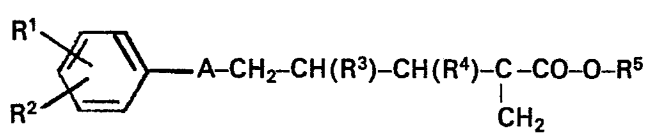

(II),

worin $R^1$, $R^2$, $R^3$, $R^4$, $R^5$ und A die oben angegebene Bedeutung haben, oxidiert und gegebenenfalls anschliessend die erhaltenen C1–C4-Niederalkylester verseift oder die erhaltenen Säuren in die Salze oder C1–C4-Niederalkylester überführt.

Die Oxidation der α-Methylencarbonsäuren II erfolgt unter den Bedingungen, wie sie dem Fachmann für die Oxidation von Kohlenstoff-Kohlenstoff-Doppelbindungen zu Epoxiden bekannt sind. Als Oxidationsmittel kommen beispielsweise Peroxoverbindungen, wie Wasserstoffperoxid, Peressigsäure, Trifluorperessigsäure, 3,5-Dinitroperbenzoesäure, bevorzugt m-Chlorperbenzoesäure oder Permaleinsäure, in Betracht. Die Reaktion wird zweckmässigerweise in inerten Lösungsmitteln, z.B. aromatischen oder chlorierten Kohlenwasserstoffen, wie Benzol, Toluol, Dichlormethan, Chloroform durchgeführt. Die Reaktionstemperaturen liegen zwischen 0° und der Siedetemperatur des Lösungsmittels, bevorzugt zwischen 20° und 70 °C.

Die Verseifung der C1–C4-Niederalkylester erfolgt in an sich bekannter Weise. Sie wird beispielsweise mit einer wässerigen oder alkoholischen (z.B. ethanolischen) Alkalimetallhydroxid-(z.B. Kaliumhydroxid-)Lösung bei Raumtemperatur vorgenommen, gegebenenfalls unter Zusatz eines inerten Verdünnungsmittels, wie Dioxan oder Toluol.

Die Überführung der Carbonsäuren der allgemeinen Formel I ($R^5$ = –H) in die Salze kann durch direkte alkalische Hydrolyse der Säurederivative I ($R^5$ = C1–C4-Niederalkyl) erfolgen. Als alkalischer Reaktionspartner wird diejenige anorganische oder organische Base verwendet, deren Salz gewünscht wird. Man erhält die Salze aber auch, indem man die Carbonsäuren I ($R^5$ = –H) mit dem stöchiometrischen Äquivalent an entsprechender Base, z.B. Natriumhydroxid oder Natriummethanolat umsetzt, oder leicht lösliche Salze durch doppelte Umsetzung in schwer lösliche Salze überführt, oder beliebige Salze in pharmakologisch verträgliche Salze überführt.

Die Überführung der Phenylalkyloxirancarbonsäuren der allgemeinen Formel I ($R^5$ = –H) in die Niederalkylester ($R^5$ = C1–C4-Niederalkyl) erfolgt in üblicher Weise. Beispielsweise werden sie mit Niederalkanolen in Gegenwart von starken Säuren, wie Schwefelsäure, p-Toluolsulfonsäure, oder von sauren Ionenaustauschern unter Bedingungen, bei denen keine Decarboxylierung stattfindet, oder mit Dialkylsulfaten oder Alkylhalogeniden in Gegenwart von Diazabicycloundecen oder Diazabicyclononen in inerten Lösungsmitteln, wie Benzol, Toluol, Aceton, verestert.

Die Verbindungen der allgemeinen Formel I fallen normalerweise in Form von Diastereometen oder Racematen an, die mittels bekannter Verfahren in die Enantiomeren getrennt werden. Die Trennung der Diastereomeren erfolgt z.B. aufgrund ihrer unterschiedlichen physikochemischen Eigenschaften, wie Schmelzpunkt, Löslichkeit; die Trennung der Racemate in die optisch-aktiven Isomeren erfolgt mit Hilfe optisch-aktiver Spaltungsmittel, z.B. optisch-aktiver Basen, wie 1- und d-1-Phenylethylamin, Cinchonidin oder d-Ephedrin, aus denen Salze der Säuren der allgemeinen Formel I, oder optisch-aktiver Alkohole, wie Borneol oder Menthol, aus denen Ester der Carbonsäuren der allgemeinen Formel I hergestellt werden. Man kann auch racemische Gemische durch Chromatographie über optisch aktive Sorptionsmittel in die optischen Isomeren zerlegen. Alternativ werden zunächst die α-Methylencarbonsäuren II mit einem optisch-aktiven Spaltungsmittel umgesetzt, z.B. Borneol oder Menthol, die erhaltenen Produkte werden zu den entsprechenden Diastereomerengemischen der Phenylalkyloxirancarbonsäureester oxydiert, aus denen dann in üblicher Weise die optischen Isomeren der Säuren I gewonnen werden.

Die Phenylalkyl-α-methylencarbonsäuren der allgemeinen Formel II können nach an sich bekannten Methoden hergestellt werden. Sie sind wertvolle Zwischenprodukte für die Synthese der Oxirancarbonsäuren I.

Die Herstellung der Phenylalkyl-α-methylencarbonsäuren II erfolgt beispielsweise in Analogie zu H. Stetter und H. Kuhlmann [Synthesis 1979, 29] durch Umsetzung von Malonsäurehalbestern der allgemeinen Formel III

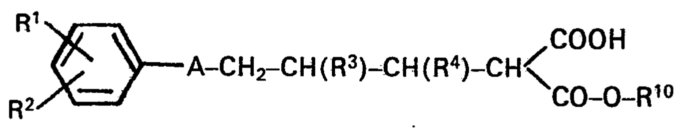

(III),

worin $R^1$, $R^2$, $R^3$, $R^4$ und A die oben angegebene Bedeutung haben und $R^{10}$ eine C1–C4-Niederalkylgruppe bedeutet, mit Formaldehyd in Pyridin in Gegenwart von sekundären Aminen, vorzugsweise Piperidin, und gegebenenfalls anschliessende Verseifung der erhaltenen C1–C4-Niederalkylester.

Die Herstellung der Malonsäurehalbester III erfolgt durch Kondensation von Oxoverbindungen IV mit Alkylidenmalonaten V und Hydrierung der erhaltenen Phenylalkylidenmalonhalbester VI nach folgendem Schema

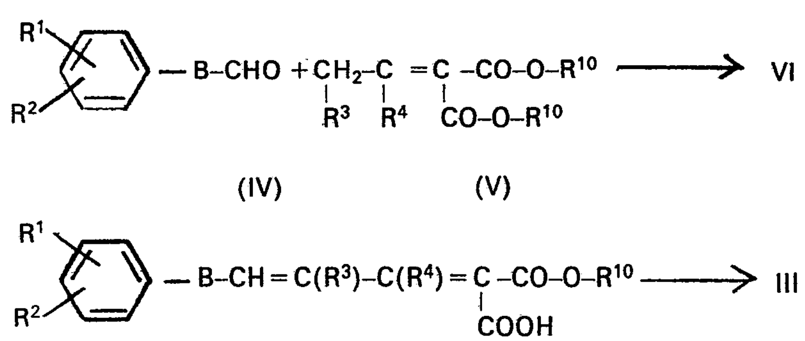

(IV) (V)

(VI)

worin $R^1$, $R^2$, $R^3$, $R^4$ und $R^{10}$ die oben angegebene Bedeutung haben und B eine Einfachbindung, eine $-CH(R^6)-CH(R^7)$-Gruppe, eine $-C(R^6)=C(R^7)$-Gruppe, eine $-CH(R^6)-CH(R^7)-CH(R^8)$-Gruppe, eine $-CH(R^6)-CH(R^7)-CH(R^8)-CH(R^9)$-Gruppe, oder eine $-C(R^6)=C(R^7)-C(R^8)=C(R^9)$-Gruppe bedeutet.

Die Kondensation der Oxoverbindungen IV mit den Alkylidenmalonaten V erfolgt unter milden Bedingungen und mit sehr guter Ausbeute. Besonders erstaunlich ist, dass Oxoverbindungen

IV, in denen B nicht eine Einfachbindung darstellt, die Kondensation ohne Weiter- bzw. Nebenreaktion durchlaufen.

Die Kondensation der Oxoverbindungen IV wird bevorzugt in Alkanolen, insbesondere in $R^{10}$–OH bei Raumtemperatur in Gegenwart molarer Mengen an Benzyltrialkylammoniumhydroxiden, worin Alkyl 1 bis 11 Kohlenstoffatome hat und bevorzugt eine Ethyl- oder Methylgruppe ist, oder Tetraalkylammoniumhydroxiden, worin Alkyl 1 bis 11 Kohlenstoffatome hat und bevorzugt eine Cetyl-, Ethyl- oder Methylgruppe ist, durchgeführt. Alternativ dazu kann man zur Kondensation bei unwesentlich niedrigeren Ausbeuten auch molare Mengen ethanolisches Natrium- oder Kaliumhydroxid in Gegenwart katalytischer Mengen an Benzyltrialkylammoniumhalogeniden verwenden, wobei intermediär das entsprechende Benzyltrialkylammoniumhydroxid gebildet wird. Selbst bei Abwesenheit katalytischer Mengen Benzyltrialkylammoniumhalogenid verläuft die Kondensation glatt und mit vernünftigen Ausbeuten. Aufarbeitung in wässriger Lösung liefert dann nach Ansäuern die entsprechenden Verbindungen VI.

Die Hydrierung der Phenylalkylidenmalonhalbester VI erfolgt nach Methoden, wie sie dem Fachmann an sich bekannt sind. Beispielsweise wird die Hydrierung mit Wasserstoff an Palladium, Platin, Rhodium oder Ruthenium, bei 0° bis 60 °C, vorzugsweise bei Raumtemperatur, unter 1–200, vorzugsweise 1–10 bar Druck und in einem inerten Lösungsmittel wie Ethanol, Essigsäureethylester, vorzugsweise $R^{10}$–OH durchgeführt.

Die folgenden Beispiele dienen zur näheren Erläuterung der Erfindung. «F» bedeutet «Schmelzpunkt»; «Kp.» bedeutet Siedepunkt. Temperaturangaben erfolgen in °C.

Beispiel 1

2-(4-Methyl -5- phenylpentyl)-oxiran -2- carbonsäureethylester

a) 2-(4-Methyl -5- phenylpentyl)-oxiran -2-carbonsäureethylester

7,80 g 6-Methyl-2-methylen-7-phenylheptansäureethylester und 12,9 g m-Chlorperbenzoesäure (85%ig) werden in 110 ml Methylenchlorid 40 Stunden unter Rückfluss gekocht. Man lässt abkühlen, filtriert von der abgeschiedenen m-Chlorbenzoesäure ab, engt das Filtrat ein, nimmt den Rückstand mit 30 ml Aceton auf, gibt 20 ml einer gesättigten Natriumcarbonatlösung hinzu und rührt 1 Stunde bei 0°. Man verdünnt mit 100 ml Wasser, extrahiert 3 mal mit je 50 ml Methylenchlorid, engt die organische Phase ein und destilliert den Rückstand. Man erhält 4,80 g der Titelverbindung vom Kp. 142–144° bei 0,008 Torr (1,06 Pa).

b) 6-Methyl -2- methylen -7- phenylheptansäureethylester

13,1 g 4-Methyl -5- phenyl-pentylmalonsäuremonoethylester, 1,26 g Paraformaldehyd, 8,2 ml Pyridin und 0,5 ml Piperidin werden 5 Stunden bei 50° gerührt. Die Reaktionsmischung wird nach dem Abkühlen mit 100 ml Wasser versetzt und 3 mal mit je 50 ml Hexan extrahiert. Die organische Phase wird nach dem Waschen mit 1 n Salzsäure, Wasser und Natriumhydrogencarbonatlösung eingeengt und destilliert. Man erhält 8,3 g 6-Methyl -2- methylen -7- phenylheptansäureethylester vom Kp. 110–114° bei 0,008 Torr (1,06 Pa).

c) 4-Methyl -5- phenylpentylmalonsäuremonoethylester

18,6 g 5-Methyl -6- phenyl-1,3,5-hexatrien-1,1-dicarbonsäuremonoethylester und 1,9 g Palladium-Kohle (5%ig) werden in einer Umlaufhydrierapparatur in 300 ml Ethanol suspendiert und bei 20° hydriert. Nach einer Wasserstoffaufnahme von 4,5 l wird eine sprunghafte Abnahme der Wasserstoffaufnahme beobachtet. Zur Aufarbeitung filtriert man vom Katalysator ab, wäscht den Filterrückstand mit 50 ml Ethanol und engt das Filtrat bei 40–50° am Rotationsverdampfer vollständig ein. Man erhält 17,5 g 4-Methyl -5-phenylpentylmalonsäuremonoethylester als viskoses Öl.

d) 5-Methyl -6- phenyl-1,3,5-hexatrien-1,1-dicarbonsäuremonoethylester

Zu 21,9 g 2-Methyl-zimtaldehyd und 30,0 g Ethylidenmalonsäurediethylester werden unter Eiskühlung und Rühren 34,5 g Benzyltriethylammoniumhydroxid, gelöst in 140 ml Ethanol, in der Weise zugetropft, dass die Innentemperatur 20° nicht übersteigt. Anschliessend rührt man weitere 20 Stunden bei 20°.

Zur Aufarbeitung werden 80 ml Ethanol im Wasserstrahlvakuum bei 40° abdestilliert, zum Rückstand 800 ml Wasser zugegeben, 2 × mit je 150 ml Diisopropylether und 2 × mit 100 ml Cyclohexan extrahiert. Anschliessend wird das Produkt durch Ansäuern der wässrigen Phase mit 1 n Salzsäure bis pH 3 unter kräftigem Rühren gefällt, abgesaugt, mit Wasser gewaschen, und bis zur Gewichtskonstanz getrocknet. Man erhält 29,4 g 5-Methyl -6- phenyl-1,3,5-hexatrien-1,1-dicarbonsäuremonoethylester als gelben Feststoff vom F. 132°.

Beispiel 2

2-(2-Ethyl -5- phenylpentyl)-oxiran -2- carbonsäureethylester

a) 2-(2-Ethyl -5- phenylpentyl)-oxiran -2-carbonsäureethylester

Nach der in Beispiel 1 a) beschriebenen Arbeitsweise erhält man aus 6,9 g 4-Ethyl -2- methylen -7- phenylheptansäureethylester und 12 g m-Chlorperbenzoesäure in 80 ml Methylenchlorid 4,5 g der Titelverbindung vom Kp. 138–141° bei 0,005 Torr (0,66 Pa).

b) 4-Ethyl -2- methylen -7- phenylheptansäureethylester

Nach der in Beispiel 1 b) beschriebenen Arbeitsweise erhält man aus 16,0 g 2-Ethyl-5-phenylpentylmalonsäuremonoethylester, 1,35 g Paraformaldehyd, 9,1 ml Pyridin und 0,6 ml Piperidin 8,9 g 4-Ethyl -2- methylen -7- phenylheptansäureethylester vom Kp. 125–128° bei 0,1 Torr (13,3 Pa).

c) 2-Ethyl -5- phenylpentylmalonsäuremonoethylester

Nach der in Beispiel 1 c) beschriebenen Arbeitsweise erhält man aus 21,3 g 3-Ethyl -6- phenyl- 1,3,5- hexatrien-1,1-dicarbonsäuremonoethylester unter Zusatz von 2,1 g Palladium-Kohle (5%ig) mit Wasserstoff in 280 ml Ethanol 21 g 2-Ethyl -5- phenylpentylmalonsäuremonoethylester als viskoses Öl. Eine Probe wird an Kieselgel neutral chromatographiert. Der $R_f$-Wert in Chloroform/Methanol (19:1) beträgt 0,58.

d) 3-Ethyl -6- phenyl-1,3,5-hexatrien-1,1-dicarbonsäuremonoethylester

Nach der in Beispiel 1 d) beschriebenen Arbeitsweise erhält man aus 26,4 g Zimtaldehyd, 50 g n-Butylidenmalonsäurediethylester und 46 g Benzyltriethylammoniumhydroxid in 180 ml Ethanol 53 g 3-Ethyl -6- phenyl-1,3,5-hexatrien-1,1-dicarbonsäuremonoethylester als Gemisch aus Öl und Feststoff. Umkristallisation aus Ethanol liefert ein Produkt vom F. 95–96° (zitronengelbe Nadeln).

Beispiel 3

2-[5-(4-Chlorphenyl) -2- ethylpentyl]-oxiran-2- carbonsäureethylester

a) 2-[5-(4-Chlorphenyl) -2- ethylpentyl]-oxiran -2- carbonsäureethylester

Nach der in Beispiel 1 a) beschriebenen Arbeitsweise erhält man aus 15,4 g 7-(4-Chlorphenyl)- 4- ethyl -2- methylen-heptansäureethylester und 22 g m-Chlorperbenzoesäure in 600 ml Methylenchlorid 11 g der Titelverbindung vom Kp. 150–155° bei 0,005 Torr (0,66 Pa).

b) 7-(4-Chlorphenyl) -4- ethyl -2- methylen-heptansäureethylester

Nach der in Beispiel 1 b) beschriebenen Arbeitsweise erhält man aus 20 g 5-(4-Chlorphenyl) -2- ethylpentylmalonsäuremonoethylester, 1,70 g Paraformaldehyd, 8 ml Pyridin und 0,9 ml Piperidin 13 g 7-(4-Chlorphenyl) -4- ethyl -2- methylenheptansäureethylester vom Kp. 130–135° bei 0,01 Torr (1,33 Pa).

c) 5-(4-Chlorphenyl) -2- ethylpentylmalonsäuremonoethylester

Nach der in Beispiel 1 c) beschriebenen Arbeitsweise erhält man aus 30 g 6-(4-Chlorphenyl) -3- ethyl-1,3,5-hexatrien-1,1-dicarbonsäuremonoethylester mit Wasserstoff in Gegenwart von 1,5 g Platin-Kohle (5%ig) in 400 ml Ethanol 29 g 5-(4-Chlorphenyl) -2- ethylpentylmalonsäuremonoethylester als viskoses Öl.

Chromatographie an Kieselgel neutral in Chloroform/Methanol (19:1) liefert einen $R_f$-Wert von 0,30.

d) 6-(4-Chlorphenyl) -3- ethyl-1,3,5-hexatrien-1,1-dicarbonsäuremonoethylester

Nach der in Beispiel 1 d) beschriebenen Arbeitsweise erhält man aus 19,2 g p-Chlorzimtaldehyd, 3,0 g n-Butylidenmalonsäurediethylester und 26 g Benzyltriethylammoniumhydroxid in 150 ml Ethanol 32 g 6-(4-Chlorphenyl) -3- ethyl- 1,3,5-hexatrien- 1,1-dicarbonsäuremonoethylester als blassgelben Feststoff vom F. 112–113°.

Beispiel 4

2-(5-Methyl -5- phenylpentyl)-oxiran -2- carbonsäureethylester

a) 2-(5-Methyl-5-phenylpentyl)- oxiran -2-carbonsäureethylester

Man gibt 17,6 g frisch zerriebenes Maleinsäureanhydrid zu einer bei 0° hergestellten Lösung von 6,0 g 85%igem Wasserstoffperoxid in 100 ml Methylenchlorid auf einmal zu, wobei die Temperatur von 0 auf –5° sinkt. Nach einstündigem Rühren im Eisbad versetzt man die Lösung von Permaleinsäure mit 17,6 g 7-Methyl -2- methylen- 7- phenylheptansäureethylester in 50 ml Methylenchlorid und erhitzt anschliessend 16 Stunden unter Rückfluss zum Sieden.

Zur Aufarbeitung filtriert man von der abgeschiedenen Maleinsäure ab, rührt zwei Stunden mit 150 ml gesättigter Natriumhydrogencarbonatlösung zuerst unter Eiskühlung, dann bei Raumtemperatur, trennt die organische Phase ab und wiederholt das Ausrühren einmal. Nach erneuter Phasentrennung wird die organische Phase mit 30 g festem Natriumhydrogensulfit gerührt (1 Stunde), (Peroxidnachweis negativ), filtriert, vollständig eingeengt und der Rückstand destilliert. Man erhält 10,5 g der Titelverbindung vom Kp. 134–138° bei 0,005 Torr (0,66 Pa).

b) 7-Methyl -2- methylen -7- phenyl-heptansäureethylester

Nach der in Beispiel 1 b) beschriebenen Arbeitsweise erhält man aus 14,6 g 5-Methyl -5-phenylpentylmalonsäuremonoethylester, 1,45 g Paraformaldehyd, 9 ml Pyridin und 0,6 ml Piperidin 9,0 g 7-Methyl -2- methylen -7- phenylheptansäureethylester vom Kp. 118–120° bei 0,1 Torr (13 Pa).

c) 5-Methyl -5- phenylpentylmalonsäuremonoethylester

Nach der in Beispiel 1 c) beschriebenen Arbeitsweise erhält man aus 25,74 g 6-Methyl -6-phenyl- 1,3,5- hexatrien- 1,1- dicarbonsäuremonoethylester mit Wasserstoff unter Zusatz von 1,3 g Palladium-Kohle (5%ig) in 500 ml Ethanol 25,5 g 5- Methyl -5- phenylpentylmalonsäuremonoethylester als viskoses Öl.

Chromatographie an Kieselgel neutral in Chloroform/Methanol (19:1) liefert einen $R_f$-Wert von 0,35.

d) 6-Methyl -6- phenyl-1,3,5-hexatrien- 1,1-dicarbonsäuremonoethylester

Nach der in Beispiel 1 d) beschriebenen Arbeitsweise erhält man aus 29,2 g 3-Methyl-zimtaldehyd, 41 g Ethylidenmalonsäurediethylester und 44,1 g Benzyltriethylammoniumhydroxid in 180 ml Ethanol 39 g 6-Methyl -6- phenyl-1,3,5-hexatrien-1,1-dicarbonsäuremonoethylester als glasigen Feststoff. Eine Probe wird an Kieselgel neutral chromatographiert. Der $R_f$-Wert in Chloroform/Methanol (19:1) beträgt 0,5.

Beispiel 5

2-(1-Methyl -5- phenylpentyl)-oxiran -2- carbonsäureethylester

a) 2-(1-Methyl -5- phenylpentyl)-oxiran -2-carbonsäureethylester

Nach der in Beispiel 1 a) beschriebenen Arbeitsweise erhält man aus 18,7 g 3-Methyl -2-methylen -7- phenylheptansäureethylester und 31 g m-Chlorperbenzoesäure in 260 ml Methylenchlorid 10,8 g der Titelverbindung vom Kp. 135–138° bei 0,005 Torr (0,66 Pa).

b) 3-Methyl -2- methylen -7- phenylheptansäureethylester

Analog der in Beispiel 1 b) beschriebenen Arbeitsweise erhält man aus 39 g 1-Methyl -5-phenylpentylmalonsäuremonoethylester, 3,8 g Paraformaldehyd, 25 ml Pyridin und 1,6 ml Piperidin 22 g 3-Methyl -2- methylen -7- phenylheptansäureethylester vom Kp. 95–100° bei 0,008 Torr (1,06 Pa).

c) 1-Methyl -5- phenylpentylmalonsäuremonoethylester

Analog der in Beispiel 1 c) beschriebenen Arbeitsweise erhält man aus 26 g 2-Methyl -6- phenyl- 1,3,5- hexatrien- 1,1- dicarbonsäuremonoethylester mit Wasserstoff in Gegenwart von 2,7 g Palladium-Kohle (5%ig) in 450 ml Ethanol 25,8 g 1-Methyl -5- phenylpentylmalonsäuremonoethylester als viskoses Öl.

d) 2-Methyl -6- phenyl-1,3,5-hexatrien-1,1-dicarbonsäuremonoethylester

Analog der in Beispiel 1 d) beschriebenen Arbeitsweise erhält man aus 36 g Zimtaldehyd, 50 g 2-Propylidenmalonsäurediethylester und 57 g Benzyltriethylammoniumhydroxid in 230 ml Ethanol 51 g 2-Methyl- 6- phenyl-1,3,5-hexatrien-1,1-dicarbonsäuremonoethylester als glasigen Feststoff.

Beispiel 6

2-(4-Methyl -5- phenylpentyl)-oxiran -2- carbonsäure

2,8 g 2-(4-Methyl -5- phenylpentyl)-oxiran-2- carbonsäureethylester werden mit 10 ml 1n Natronlauge 2 Stunden bei Raumtemperatur in 10 ml Tetrahydrofuran gerührt. Man engt auf die Hälfte ein, extrahiert zweimal mit 10 ml Diethylether, säuert mit 2n Salzsäure auf pH 3 an, extrahiert 2 × mit 20 ml Diethylether, trocknet über Natriumsulfat und engt vollständig ein. Man erhält 2,3 g der Titelverbindung als farbloses Öl.

Beispiel 7

Natrium -2- (4-methyl -5- phenylpentyl)-oxiran -2- carboxylat

2,8 g 2-(4-Methyl -5- phenylpentyl)-oxiran-2- carbonsäureethylester werden mit 10 ml 1n Natronlauge und 10 ml Ethanol 1 Stunde bei Raumtemperatur gerührt. Man engt im Vakuum ein, wobei die Titelverbindung als farbloses, glasiges Pulver zurückbleibt; Ausbeute 2,75 g.

Beispiel 8

2-[3-(4-Chlorphenyl) -2- ethylpropyl]-oxiran- 2- carbonsäureethylester

a) 2-[3-(4-Chlorphenyl) -2- ethylpropyl]-oxiran -2- carbonsäureethylester

Nach der in Beispiel 4 a) beschriebenen Arbeitsweise erhält man aus 13,5 g 5-(4-Chlorphenyl)- 4-ethyl-2-methylenvaleriansäureethylester, 4,6 g 85%igem Wasserstoffperoxid und 13,8 g Maleinsäureanhydrid in 70 ml Methylenchlorid 9 g der Titelverbindung vom Kp. 115–120° bei 0,005 Torr (0,66 Pa).

b) 5-(4-Chlorphenyl) -4- ethyl -2- methylenvaleriansäureethylester

Nach der in Beispiel 1 b) beschriebenen Arbeitsweise erhält man aus 21 g 3-(4-Chlorphenyl) -2- ethylpropylmalonsäuremonoethylester, 2,05 g Paraformaldehyd, 13 ml Pyridin und 1 ml Piperidin 16 g 5-(4-Chlorphenyl) -4- ethyl -2-methylenvaleriansäureethylester vom Kp. 135–137° bei 0,05 Torr (6,65 Pa).

c) 3-(4-Chlorphenyl) -2- ethylpropylmalonsäuremonoethylester

Nach der in Beispiel 1 c) beschriebenen Arbeitsweise erhält man aus 30 g 4-(4-Chlorphenyl) -3- ethyl- 1,3- butadien- 1,1- dicarbonsäuremonoethylester mit Wasserstoff in Gegenwart von 1,5 g Platin-Kohle (5%ig) in 300 ml Ethanol 30 g 3-(4-Chlorphenyl) -2- ethylpropylmalonsäuremonoethylester als viskoses Öl.

d) 4-(4-Chlorphenyl) -3- ethyl- 1,3- butadien-1,1-dicarbonsäuremonoethylester

Nach der in Beispiel 1 d) beschriebenen Arbeitsweise erhält man aus 44 g 4-Chlorbenzaldehyd, 65 g Butylidenmalonsäurediethylester und 68 g Benzyltriethylammoniumhydroxid in 400 ml Ethanol 73 g 4-(4-Chlorphenyl) -3- ethyl-1,3-butadien-1,1-dicarbonsäuremonoethylester als hochviskoses, gelbes Öl.

Beispiel 9

2-[1-Methyl -3- (3-trifluormethylphenyl)-propyl]-oxiran -2- carbonsäureethylester

a) 2-[1-Methyl -3- (3-trifluormethylphenyl)-propyl]-oxiran -2- carbonsäureethylester

Nach der in Beispiel 1 a) beschriebenen Arbeitsweise erhält man aus 16 g 3-Methyl -2-methylen- 5- (3-trifluormethylphenyl)- valeriansäureethylester und 21,5 g m-Chlorperbenzoesäure in 160 ml Methylenchlorid 9,4 g der Titelverbindung vom Kp. 111–112° bei 0,05 Torr (6,65 Pa).

b) 3-Methyl -2- methylen -5- (3-trifluormethylphenyl)-valeriansäureethylester

Nach der in Beispiel 1 b) beschriebenen Arbeitsweise erhält man aus 25,5 g 1-Methyl -3-(3-trifluormethylphenyl)-propylmalonsäuremonoethylester, 2,95 g Paraformaldehyd, 25 ml Pyridin und 1,6 ml Piperidin 20,1 g 3-Methyl -2- methylen- 5- (3-trifluormethylphenyl)-valeriansäureethylester vom Kp. 100–104° bei 0,05 Torr (6,65Pa).

c) 1-Methyl -3- (3-trifluormethylphenyl)-propylmalonsäuremonoethylester

Nach der in Beispiel 1 c) beschriebenen Arbeitsweise erhält man aus 52 g 2-Methyl -4-(3-trifluormethylphenyl)- 1,3-butadien- 1,1- dicarbonsäuremonoethylester mit Wasserstoff in Gegenwart von 2,6 g Palladium-Kohle (5%ig) in 400 ml Ethanol 51 g 1-Methyl -3- (3-trifluormethylphenyl)-propylmalonsäuremonoethylester als viskoses Öl.

d) 2-Methyl -4- (3-trifluormethylphenyl)-1,3-butadien-1,1-dicarbonsäuremonoethylester

Nach der in Beispiel 1 d) beschriebenen Arbeitsweise erhält man aus 52,2 g 3-Trifluormethylbenzaldehyd, 66 g 2-Propylidenmalonsäurediethylester und 64 g Benzyltrimethylammoniumhydroxid in 700 ml Ethanol 62 g 2-Methyl -4- (3-trifluormethylphenyl)-1,3-butadien-1,1-dicarbonsäuremonoethylester.

Beispiel 10

6-(4-Chlorphenyl)-1,3,5-hexatrien-1,1-dicarbonsäuremonoethylester

a) Zu 66,65 g (0,4 mol) 4-Chlorzimtaldehyd und 76,5 g (0,42 mol) Ethylidenmalonsäurediethylester werden unter Eiskühlung und Rühren 400 ml einer 1,1 molaren Lösung (0,44 mol) von Benzyltriethylammoniumhydroxid (≙ 92,4 g) in Ethanol in der Weise zugetropft, dass die Innentemperatur 20° nicht übersteigt. Anschliessend rührt man 20 Stunden bei 20° nach.

Zur Aufarbeitung werden 200 ml Ethanol im Wasserstrahlvakuum bei 40° abdestilliert, zum Rückstand 2 l Wasser zugegeben, 2 × mit je 400 ml Diisopropylether und 2 × mit 200 ml Cyclohexan extrahiert. Anschliessend wird das Produkt durch Ansäuern der wässrigen Phase mit 1 n Salzsäure bis pH 3 unter kräftigen Rühren als gelbroter Feststoff gefällt, abgesaugt, mit Wasser gewaschen und bis zur Gewichtskonstanz getrocknet. Das erhaltene Rohprodukt (103 g, 84% der Theorie) wird ohne weitere Reinigung gemäss Beispiel 10 d) weiterverwendet.

Umfällen aus 600 g Ethanol mit 1200 g Wasser liefert 91 g der Titelverbindung vom F. 138–145° (gelber Feststoff).

Chromatographie an Kieselgel mit Chloroform/Methanol (19:1) liefert einen Rf-Wert von 0,33.

b) Alternativ wird die Titelverbindung wie folgt erhalten:

Zu einer Lösung von 16,6 g 4-Chlorzimtaldehyd (0,1 mol), 20 g (0,11 mol) Ethylidenmalonsäurediethylester und 4,5 g (0,02 mol) Benzyltriethylammoniumchlorid in 100 ml Ethanol tropft man unter Eiskühlung eine Lösung von 4,0 g (0,1 mol) Natriumhydroxid in 100 ml Ethanol, rührt 24 Stunden nach und arbeitet analog Beispiel 10a) auf. Man erhält 23,1 g (75% der Theorie) 6-(4-Chlorphenyl)-1,3,5-hexatrien-1,1-dicarbonsäuremonoethylester.

c) Alternativ wird die Titelverbindung wie folgt erhalten:

Zu einer Lösung von 16,6 g 4-Chlorzimtaldehyd (0,1 mol) und 20 g (0,11 mol) Ethylidenmalonsäurediethylester in 100 ml Ethanol tropft man unter Eiskühlung eine Lösung von 4,0 g (0,1 mol) Natriumhydroxid in 100 ml Ethanol, rührt 24 Stunden und arbeitet analog Beispiel 10 a) auf. Man erhält 20,7 g (68% der Theorie) 6-(4-Chlorphenyl)- 1,3,5-hexatrien- 1,1-dicarbonsäuremonoethylester.

d) Alternativ wird die Titelverbindung wie folgt erhalten:

Zu 400 g (2,4 mol) 4-Chlorzimtaldehyd und 466 g (2,5 mol) Ethylidenmalonsäurediethylester in 0,4 l trockenem Ethanol tropft man bei –5 bis +3° innerhalb von 7 Stunden 2,8 l einer 1-molaren Lösung von Tetramethylammoniumhydroxid [hergestellt in Anlehnung an die Herstellungsvorschrift für Benzyltrimethylammoniumethoxid in Organic Synthesis IV (1963), 98] in Ethanol. Man rührt weitere 20 Stunden, destilliert im Wasserstrahlvakuum 2 l Ethanol ab und arbeitet nach der in Beispiel 10 a) beschriebenen Arbeitsweise auf. Man erhält 588 g der Titelverbindung vom F. 136–138°.

e) 103 g 6-(4-Chlorphenyl)-1,3,5-hexatrien-1,1-dicarbonsäuremonoethylester und 5 g Platin-Kohle (5%ig) werden in einer Umlaufhydrierapparatur in 1,2 l Ethanol suspendiert und bei 20° hydriert. Nach einer Wasserstoffaufnahme von 23 l wird eine sprunghafte Abnahme der Wasserstoffaufnahme beobachtet. Zur Aufarbeitung filtriert man vom Katalysator ab, wäscht den Filterrückstand mit 100 ml Ethanol und engt das Filtrat bei 40–50° am Rotationsverdampfer vollständig ein. Man erhält 103 g 5-(4-Chlorphenyl)-pentylmalonsäuremonoethylester als viskoses Öl. Eine Probe wird an Kieselgel neutral mit Chloroform/Methanol (19:1) chromatographiert. Der Rf-Wert beträgt 0,30.

Beispiel 11

6-Phenyl-1,3,5-hexatrien-1,1-dicarbonsäuremonoethylester

a) Nach der in Beispiel 10 a) beschriebenen Arbeitsweise erhält man aus 34 g Zimtaldehyd, 52,3 g Ethylidenmalonsäurediethylester und 63 g Benzyltriethylammoniumhydroxid in 300 ml Ethanol 38.8 g 6-Phenyl-1,3,5-hexatrien-1,1-dicarbonsäuremonoethylester vom F. 139–142° (orangegelbe Nadeln).

b) Nach der in Beispiel 10 c) beschriebenen Arbeitsweise erhält man durch Hydrierung von 50 g 6-Phenyl-1,3,5-hexatrien-1,1-dicarbonsäuremonoethylester an 2,5 g Palladium-Kohle (5%ig) in 600 ml Ethanol 50,5 g 5-Phenylpentylmalonsäuremonoethylester als viskoses Öl.

Beispiel 12

6-Phenyl- 1,3-hexadien- 1,1-dicarbonsäuremonoethylester

a) Nach der in Beispiel 10 a) beschriebenen Arbeitsweise erhält man aus 13,4 g 3-Phenylpropionaldehyd, 20 g Ethylidenmalonsäurediethylester und 16,5 g Tetraethylammoniumhydroxid in 190 ml Ethanol 15,8 g der Titelverbindung als gelbes, viskoses Öl.

b) Nach der in Beispiel 10 c) beschriebenen Arbeitsweise erhält man aus 10 g 6-Phenyl-1,3-hexadien-1,1-dicarbonsäuremonoethylester mit Wasserstoff in Gegenwart von 0,5 g Palladium-Kohle (5%ig) in 80 ml Ethanol 9,9 g 5-Phenylpentylmalonsäuremonoethylester als viskoses Öl. Die Verbindung ist laut DC, NMR und IR identisch mit der nach Beispiel 11b) erhaltenen Verbindung.

Beispiel 13

2-(2-Ethyl -3- phenylpropyl)-oxiran -2- carbonsäureethylester

a) 2-(2-Ethyl -3- phenylpropyl)-oxiran -2-carbonsäureethylester

Nach der in Beispiel 4 a) beschriebenen Arbeitsweise erhält man aus 18,5 g 4-Ethyl -5- phenyl- 2- methylenvaleriansäureethylester, 7,5 g 85%igem Wasserstoffperoxid und 22,5 g Maleinsäureanhydrid in 100 ml Methylenchlorid 14 g der Titelverbindung vom Kp. 110–112° bei 0,005 Torr (0,66 Pa).

b) 4-Ethyl -5- phenyl -2- methylenvaleriansäureethylester

Nach der in Beispiel 1 b) beschriebenen Arbeitsweise erhält man aus 55,6 g 2-Ethyl -3- phenylpropylmalonsäuremonoethylester, 6,0 g Paraformaldehyd, 37 ml Pyridin und 2 ml Piperidin 42 g 4-Ethyl -5- phenyl -2- methylenvaleriansäureethylester vom Kp. 95–98° bei 0,05 Torr (6,65 Pa).

c) 2-Ethyl -3- phenylpropylmalonsäuremonoethylester

Nach der in Beispiel 1 c) beschriebenen Arbeitsweise erhält man aus 69 g 3-Ethyl -4- phenyl- 1,3-butadien- 1,1-dicarbonsäuremonoethylester mit Wasserstoff in Gegenwart von 3,5 g Palladium-Kohle (5%ig) in 700 ml Ethanol 70 g 2-Ethyl- 3- phenylpropylmalonsäuremonoethylester als viskoses Öl.

d) 3-Ethyl -4- phenyl-1,3-butadien-1,1-dicarbonsäuremonoethylester

Nach der in Beispiel 10 b) beschriebenen Arbeitsweise erhält man aus 53 g Benzaldehyd, 107 g n-Butylidenmalonsäurediethylester, 11,4 g Benzyltriethylammoniumchlorid und 20 g Natriumhydroxid in 600 ml Ethanol 93 g 3-Ethyl -4- phenyl-1,3-butadien-1,1-dicarbonsäuremonoethylester (orangegelbes, viskoses Öl).

Beispiel 14

2-(4-Methyl -7- phenylheptyl)-oxiran -2- carbonsäureethylester

a) 2-(4-Methyl -7- phenylheptyl)-oxiran -2-carbonsäureethylester

Nach der in Beispiel 4 a) beschriebenen Arbeitsweise erhält man aus 8,0 g 6-Methyl -9-phenyl -2- methylennonansäureethylester, 2,78 g 85%igem Wasserstoffperoxid und 8,15 g Maleinsäureanhydrid in 60 ml Methylenchlorid 1,5 g der Titelverbindung vom Kp. 150 °C bei 0,01 Torr (1,3 Pa).

b) 6-Methyl -9- phenyl -2- methylennonansäureethylester

Nach der in Beispiel 1 b) beschriebenen Arbeitsweise erhält man aus 25 g 4-Methyl -7- phenylheptylmalonsäuremonoethylester, 2,34 g Paraformaldehyd, 14,3 ml Pyridin und 0,8 ml Piperidin 9,4 g 6-Methyl -9- phenyl -2- methylennonansäureethylester.

c) 4-Methyl -7- phenylheptylmalonsäuremonoethylester

Nach der in Beispiel 1 c) beschriebenen Arbeitsweise erhält man aus 28 g 5-Methyl -8- phenyl-1,3,5,7- octatetraen- 1,1- dicarbonsäuremonoethylester mit Wasserstoff in Gegenwart von 3,0 g Palladium-Kohle (5%ig) in 300 ml Essigsäureethylester 25 g 4-Methyl -7- phenylheptylmalonsäuremonoethylester als gelbes Öl.

d) 5-Methyl -8- phenyl-1,3,5,7-octatetraen-1,1-dicarbonsäuremonoethylester

Nach der in Beispiel 10 a) beschriebenen Arbeitsweise erhält man aus 24,0 g 2-Methyl -5-phenyl-2,4-pentadienal, 31,1 g Ethylidenmalonsäurediethylester und 365 ml einer 0,5 molaren Lösung (0,182 mol) von Benzyltrimethylammoniumhydroxid in Ethanol 28 g 5-Methyl -8- phenyl- 1,3,5,7-octatetraen- 1,1-dicarbonsäuremonoethylester, der nach dem Ansäuern mit Salzsäure als orangerotes Öl anfällt.

e) 2-Methyl -5-phenyl-2,4-pentadienal

Literatur: M. Lipp, F. Dallacker, Chem. Ber. 90, 1730 (1957).

Beispiel 15

2-[5-(4-Chlorphenyl) -4- methylpentyl]-oxiran -2- cabonsäureethylester

a) 2-[5-(4-Chlorphenyl) -4- methylpentyl]-oxiran -2- carbonsäureethylester

Nach der in Beispiel 4 a) beschriebenen Arbeitsweise erhält man aus 12,3 g 7-(4-Chlorphenyl) -6- methyl -2- methylenheptansäureethylester, 4,17 g 85%igem Wasserstoffperoxid und 12,27 g Maleinsäureanhydrid in 70 ml Methylenchlorid 10,9 g der Titelverbindung vom Kp. 200–202 °C bei 0,01 Torr (1,3 Pa).

b) 7-(4-Chlorphenyl) -6- methyl -2- methylenheptansäureethylester

Nach der in Beispiel 1 b) beschriebenen Arbeitsweise erhält man aus 21,45 g 5-(4-Chlorphenyl) -4- methylpentylmalonsäuremonoethylester, 1,82 g Paraformaldehyd, 12,74 ml Pyridin und 0,96 ml Piperidin 15,5 g 7-(4- Chlorphenyl) -6- methyl -2- methylenheptansäureethylester als hellgelbes Öl.

c) 5-(4-Chlorphenyl) -4- methylpentylmalonsäuremonoethylester

Nach der in Beispiel 1 c) beschriebenen Arbeitsweise erhält man aus 23,0 g 6-(4-Chlorphenyl) -5- methyl-1,3,5-hexatrien-1,1-dicarbonsäuremonoethylester mit Wasserstoff in Gegenwart von 1,2 g Platin-Kohle (5%ig) in 400 ml Essigsäureethylester 25,0 g 5-(4-Chlorphenyl) -4-methylpentylmalonsäuremonoethylester als farbloses Öl.

d) 6-(4-Chlorphenyl) -5- methyl-1,3,5-hexatrien-1,1-dicarbonsäuremonoethylester

Nach der in Beispiel 1 d) beschriebenen Arbeitsweise erhält man aus 47 g 3-(4-Chlorphenyl) -2- methyl-propenal, 51,9 g Ethylidenmalonsäurediethylester und 48,2 g Benzyltrimethylammoniumhydroxid in 400 ml Ethanol 53,8 g 6-(4-Chlorphenyl) -5- methyl-1,3,5-hexatrien-1,1-dicarbonsäuremonoethylester als orangeroten Feststoff vom F. 151–153 °C (aus Ethanol).

Beispiel 16

2-(6-Methyl -7- phenylheptyl)-oxiran -2- carbonsäureethylester

a) 2-(6-Methyl -7- phenylheptyl)-oxiran -2-carbonsäureethylester

Nach der in Beispiel 4 a) beschriebenen Arbeitsweise erhält man aus 15 g 8-Methyl -9- phenyl -2- methylennonansäureethylester, 4,42 g 85%igem Wasserstoffperoxid und 15,3 g Maleinsäureanhydrid in 130 ml Methylenchlorid 8,7 g der Titelverbindung vom Kp. 135–144 °C bei 0,01 Torr (1,3 Pa).

b) 8-Methyl -9- phenyl -2- methylennonansäureethylester

Nach der in Beispiel 1 b) beschriebenen Arbeitsweise erhält man aus 29,5 g 6-Methyl -7-phenylheptylmalonsäuremonoethylester, 3,46 g Paraformaldehyd, 60 ml Pyridin und 5 ml Piperidin 19,1 g 8-Methyl -9- phenyl -2- methylennonansäureethylester vom Kp. 140 °C bei 0,01 Torr (1,3 Pa).

c) 6-Methyl -7- phenylheptylmalonsäuremonoethylester

Nach der in Beispiel 1 c) beschriebenen Arbeitsweise erhält man aus 31,6 g 7-Methyl -8-phenyl- 1,3,5,7-octatetraen- 1,1-dicarbonsäuremonoethylester mit Wasserstoff in Gegenwart von 3,0 g Palladium-Kohle (5%ig) in 300 ml Essigsäureethylester 30,5 g 6-Methyl -7- phenylheptylmalonsäuremonoethylester.

d) 7-Methyl -8- phenyl-1,3,5,7-octatetraen-1,1-dicarbonsäuremonoethylester

Nach der in Beispiel 10 a) beschriebenen Arbeitsweise erhält man aus 8,6 g 4-Methyl -5-phenyl-2,4-pentadienal, 10,2 g Ethylidenmalonsäurediethylester und 78 ml einer 0,8 molaren Lösung von Tetramethylammoniumhydroxid in 30 ml Ethanol 10,1 g 7-Methyl -8- phenyl-1,3,5,7-octatetraen-1,1-dicarbonsäuremonoethylester vom F. 137–144 °C.

e) 4-Methyl -5- phenyl-2,4-pentadienal

Hergestellt analog 3-(5-Brom -2- methoxyphenyl)-propenal [J.H. Billman, J.A. Tonnis, Journal of Pharm. Science 60, 1188 (1971)].

Galenische Beispiele

Beispiel 1

Ansatz für Ampullen

100 g 2-(4-Methyl -5- phenylpentyl)-oxiran-2- carbonsäure werden in ca. 8 Liter aqua bidest. unter Zusatz der äquivalenten Menge Natronlauge gelöst. Die Lösung wird auf pH 7,0 ± 0,5 eingestellt und mit aqua bidest. auf 10 Liter aufgefüllt. Dann wird steril filtriert und unter keimfreien Bedingungen in 2 ml-Ampullen abgefüllt.

Beispiel 2

1000 Kapseln mit einem Wirkstoffgehalt von 25 mg werden wie folgt hergestellt:

25 g 2-[5-(4-Chlorphenyl) -4- methylpentyl]-oxiran -2- carbonsäureethylester werden in 100 ml Methylenchlorid gelöst. Die Lösung wird mit 75 g mikronisierter Kieselsäure gut gemischt. Die Mischung wird zur Trockne eingedampft und dann in Hartgelatinekapseln abgefüllt.

Beispiel 3

Tabletten mit einem Wirkstoffgehalt von 25 mg werden wie folgt hergestellt:

1,0 kg Natrium -2- (4-Methyl -7- phenylheptyl)-oxiran -2- carboxylat, 4,5 kg Xylit und 3,0 kg Calciumphosphat werden mit 0,25 kg Polyvinylpyrrolidon (MG 25 000; MG = Molekulargewicht) in ungefähr 0,5 l Wasser granuliert. Das Granulat wird durch ein Sieb von 1,25 mm Maschenweite gesiebt und nach dem Trocknen werden 0,9 kg Carboxymethylcellulose, 0,25 kg Talkum und 0,1 kg Magnesiumstearat zugegeben. Man verpresst das trockene Granulat zu Tabletten von 8 mm Durchmesser, 250 mg Gewicht und einer Härte von 5–6 kg.

Pharmakologie

Die erfindungsgemässen Phenylalkyloxirancarbonsäuren der allgemeinen Formel I senken den Blutglucosespiegel und den Blutspiegel der Ketonkörper, wobei sie sich in ihrer chemischen Struktur und ihrer Wirkungsweise grundlegend von pankreaswirksamen, betacytotropen Substanzen (z.B. Sulfonylharnstoffen) durch ihre extrapankreatische Wirkung unterscheiden und extrapankreatisch wirkenden Handelspräparaten (z.B. Buformin und Phenformin) überlegen erweisen. Ausserdem zeichnen sie sich durch ihre Langzeitwirkung aus.

In der anschliessenden Tabelle werden die untersuchten Verbindungen durch eine laufende Nummer gekennzeichnet, die wie folgt zuzuordnen ist:

| Lfd. Nr. | Name der Verbindung |
|---|---|
| 1 | Buformin |
| 2 | Phenformin |
| 3 | 2-(4-Methyl -5- phenylpentyl)-oxiran -2- carbonsäureethylester |
| 4 | 2-(2-Ethyl -5- phenylpentyl)-oxiran-2- carbonsäureethylester |
| 5 | 2-[5-(4-Chlorphenyl) -4- methylpentyl]-oxiran -2- carbonsäureethylester |
| 6 | 2-(4-Methyl -7- phenylheptyl)-oxiran -2- carbonsäureethylester |
| 7 | 2-(5-Methyl -5- phenylpentyl)-oxiran -2- carbonsäureethylester |

In Tabelle I werden Untersuchungen des Einflusses von Vertretern der erfindungsgemässen Verbindungen auf die Blutglucosekonzentration nüchterner, stoffwechselgesunder Ratten wiedergegeben. Spalte A gibt jeweils die maximale Senkung der Blutglucosekonzentration nüchterner Ratten (in % bezogen auf die Kontrollgruppe) an, die im Laufe von 6 Stunden nach einmaliger oraler Applikation von 0,6 mmol Substanz/kg Körpergewicht beobachtet wird. In der Spalte B werden Daten zur akuten Toxizität ($LD_{50}$; Maus p.o.) wiedergegeben.

Tabelle I

| Verb. Nr. | Senkung der Blutglucose-konzentration (in %) in vivo A | Akute Toxizität Maus p.o. $LD_{50}$ (mg/kg) B |
|---|---|---|
| 1 | 8 | 475 |
| 2 | 6 | 410* |
| 3 | 45 | >1 000 |
| 4 | 25 | – |
| 5 | 70 | ~ 500 |
| 6 | 35 | > 500 |
| 7 | 22 | – |

* Zit. Blickens, D.A., Riggi, S.J.: Toxicol. Appl. Pharmacol. 14 (1969) 393–400.

Die pharmakologischen Eigenschaften werden nach folgenden Methoden ermittelt:

1. Blutglucosebestimmung nach einmaliger oraler Applikation

Zur Verwendung kommen junge männliche Sprague-Dawley-Ratten (Körpergewicht 150–200 g). Die Haltung der Tiere (6 Tiere pro Dosis) erfolgt in Makrolon-Käfigen mit bis zu 4 Tieren pro Käfig (Raumtemperatur 23 °C, rel. Luftfeuchtigkeit 55%, fester Tag/Nacht-Rythmus (12/12= h) Standarddiät Altromin®). Den Ratten wird 18 Stunden vor der 1. Blutentnahme das Futter entzogen. Wasseraufnahme erfolgt ad libitum. Blutentnahmen erfolgen unmittelbar vor und 2, 4 und 6 Stunden nach Substanzgabe durch Punktion aus dem retroorbitalen Plexus. Nach Enteiweissung mit Perchlorsäure wird die Blutglucosebestimmung mittels der bei Banauch et.al. [J.Clin.Chem.Clin.Biochem. 13, 101 (1975)] beschriebener Glucose-Dehydrogenase-Methode mit Hilfe von kommerziellen Reagentien durchgeführt. Zum Vergleich wird jeweils eine mit reinem Lösungsmittel behandelte Kontrollgruppe mituntersucht (10 Tiere pro Kontrollgruppe).

2. Bestimmung der Toxizität

Die Toxizitätsuntersuchungen werden an weiblichen NMRI-Mäusen (Körpergewicht 22–26 g) durchgeführt. Die Tiere (5 Tiere pro Dosis) erhalten 18 Stunden vor der Behandlung das Futter (Altromin®) auf 50 g/50 Tiere reduziert und Wasser ad libitum. Verschiedene Dosen der Substanzen werden oral mittels Schlundsonde verabreicht (Volumen 10 ml/kg). Die Beobachtungsdauer beträgt 7 Tage. Die $LD_{50}$, d.h. die Dosis, bei der 50% der Tiere sterben, wird aus der Dosiswirkungskurve graphisch ermittelt.

**Patentansprüche für die Vertragsstaaten BE, CH, DE, FR, GB, IT, LI, LU, NL, SE**

1. Phenylalkyloxirancarbonsäuren der allgemeinen Formel I

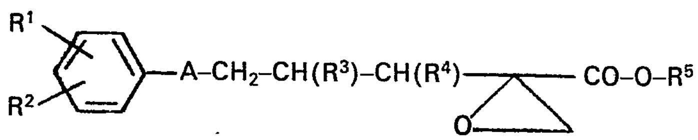

(I),

worin

$R^1$ ein Wasserstoffatom, ein Halogenatom, eine C1–C4-Niederalkylgruppe, eine C1–C4-Niederalkoxygruppe oder eine Trifluormethylgruppe bedeutet,

$R^2$ eine der Bedeutungen von $R^1$ hat,

A eine Einfachbindung, eine $-CH(R^6)-CH(R^7)$-Gruppe, eine $-CH(R^6)-CH(R^7)-CH(R^8)$-Gruppe oder eine $-CH(R^6)-CH(R^7)-CH(R^8)-CH(R^9)$-Gruppe bedeutet,

$R^5$ ein Wasserstoffatom oder eine C1–C4-Niederalkylgruppe bedeutet, einer der Substituenten $R^3$, $R^4$, $R^6$, $R^7$, $R^8$ oder $R^9$ eine C1–C4-Niederalkylgruppe darstellt und die anderen ein Wasserstoffatom bedeuten, und die Salze der Carbonsäuren.

2. Phenylalkyloxirancarbonsäuren der allgemeinen Formel I nach Anspruch 1, worin $R^1$ und $R^2$ meta- oder para-Substituenten sind und $R^1$ ein Wasserstoffatom, ein Chloratom, eine Methylgruppe oder eine Trifluormethylgruppe, $R^2$ ein Wasserstoffatom oder ein Chloratom, A eine Einfachbindung, eine $-CH(R^6)-CH(R^7)$-Gruppe oder eine $-CH(R^6)-CH(R^7)-CH(R^8)$-Gruppe bedeutet, $R^5$ ein Wasserstoffatom oder eine C1–C4-Niederalkylgruppe, einer der Substituenten $R^3$, $R^4$, $R^6$, $R^7$ oder $R^8$ eine Methyl- oder Ethylgruppe darstellt und die anderen Wasserstoffatome bedeuten.

3. Phenylalkyloxirancarbonsäuren der allgemeinen Formel I nach Anspruch 1, worin $R^1$ und $R^2$ meta- oder para-Substituenten sind und $R^1$ ein Wasserstoffatom, ein Chloratom oder eine Trifluormethylgruppe, $R^2$ ein Wasserstoffatom, A eine Einfachbindung oder eine $-CH(R^6)-CH(R^7)$-Gruppe bedeutet, $R^5$ ein Wasserstoffatom, eine Methylgruppe oder eine Ethylgruppe bedeutet, einer der Substituenten $R^3$, $R^4$, $R^6$ oder $R^7$ eine Methylgruppe darstellt und die anderen Wasserstoffatome bedeuten, und die pharmakologisch verträglichen Salze der Carbonsäuren mit anorganischen und organischen Basen.

4. Phenylalkyloxirancarbonsäuren der allgemeinen Formel I nach Anspruch 1, worin $R^1$ und $R^2$ meta- oder para-Substituenten sind, $R^1$ ein Wasserstoffatom, ein Chloratom oder eine Trifluormethylgruppe, $R^2$ ein Wasserstoffatom, A eine Einfachbindung, eine $-CH(R^6)-CH(R^7)$- oder $-CH(R^6)-CH(R^7)-CH(R^8)-CH(R^9)$-Gruppe, $R^5$ ein Wasserstoffatom oder eine Alkylgruppe mit 1 bis 4 Kohlenstoffatomen bedeutet, einer der Substituenten $R^3$, $R^4$, $R^6$, $R^7$, $R^8$ oder $R^9$ eine Methyl- oder Ethylgruppe und die anderen Wasserstoffatome darstellen.

5. Phenylalkyloxirancarbonsäuren der allgemei-

nen Formel I nach Anspruch 1, worin $R^1$ ein para-Substituent ist und ein Wasserstoffatom oder Chloratom darstellt, $R^2$ ein Wasserstoffatom, A eine $-CH(R^6)-CH(R^7)-$ oder $-CH(R^6)-CH(R^7)-CH(R^8)-CH(R^9)$-Gruppe, $R^5$ ein Wasserstoffatom oder eine Methyl- oder Ethylgruppe, $R^4$ ein Wasserstoffatom, $R^8$ ein Wasserstoffatom bedeutet, einer der Substituenten $R^3$, $R^6$, $R^7$ oder $R^9$ eine Methyl- oder Ethylgruppe und die anderen Wasserstoffatome darstellen, und die pharmakologisch verträglichen Salze der Carbonsäuren.

6. Verfahren zur Herstellung von Verbindungen nach Anspruch 1, dadurch gekennzeichnet, dass man Phenylalkyl -2- methylencarbonsäuren der allgemeinen Formel II

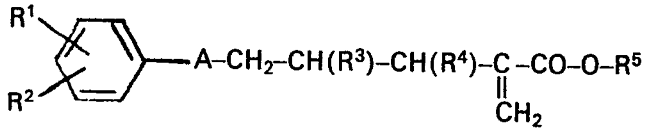

(II),

worin $R^1$, $R^2$, $R^3$, $R^4$, $R^5$ und A die in Anspruch 1 angegebene Bedeutung haben, oxidiert und gegebenenfalls anschliessend die erhaltenen C1–C4-Niederalkylester verseift oder die erhaltenen Säuren in die Salze oder C1–C4-Niederalkylester überführt.

7. Arzneimittel enthaltend eine oder mehrere Verbindungen nach einem oder mehreren der Ansprüche 1 bis 5.

8. Verbindungen nach einem oder mehreren der Ansprüche 1 bis 5 zur Anwendung in der Behandlung und Prophylaxe von Krankheiten, die auf Störungen des Glucose- und Fettstoffwechsels beruhen.

**Patentansprüche für den Vertragsstaat AT**

1. Verfahren zur Herstellung von Phenylalkyloxirancarbonsäuren der allgemeinen Formel I

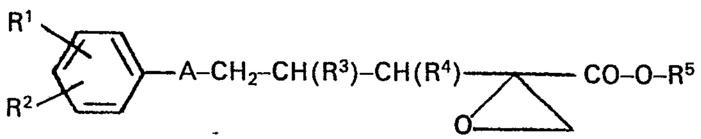

(I),

worin

$R^1$ ein Wasserstoffatom, ein Halogenatom, eine C1–C4-Niederalkylgruppe, eine C1–C4-Niederalkoxygruppe oder eine Trifluormethylgruppe bedeutet,

$R^2$ eine der Bedeutungen von $R^1$ hat,

A eine Einfachbindung, eine $-CH(R^6)-CH(R^7)$-Gruppe, eine $-CH(R^6)-CH(R^7)-CH(R^8)$-Gruppe oder eine $-CH(R^6)-CH(R^7)-CH(R^8)-CH(R^9)$-Gruppe bedeutet,

$R^5$ ein Wasserstoffatom oder eine C1–C4-Niederalkylgruppe bedeutet, einer der Substituenten $R^3$, $R^4$, $R^6$, $R^7$, $R^8$ oder $R^9$ eine Niederalkylgruppe darstellt und die anderen ein Wasserstoffatom bedeuten, und den Salzen der Carbonsäuren, dadurch gekennzeichnet, dass man Phenylalkyl -2- methylencarbonsäuren der allgemeinen Formel II

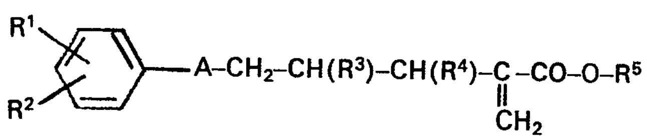

(II),

worin $R^1$, $R^2$, $R^3$, $R^4$, $R^5$ und A die in Anspruch 1 angegebene Bedeutung haben, oxidiert und gegebenenfalls anschliessend die erhaltenen C1–C4-Niederalkylester verseift oder die erhaltenen Säuren in die Salze oder C1–C4-Niederalkylester überführt.

2. Verfahren nach Anspruch 1 zur Herstellung von Phenylalkyloxirancarbonsäuren der allgemeinen Formel I, dadurch gekennzeichnet, dass man Phenylalkyl -2- methylencarbonsäuren der allgemeinen Formel II oxidiert und gegebenenfalls anschliessend die erhaltenen C1–C4-Niederalkylester verseift oder die erhaltenen Säuren in die Salze oder C1–C4-Niederalkylester überführt, wobei $R^1$ und $R^2$ meta- oder para-Substituenten sind und $R^1$ ein Wasserstoffatom, ein Chloratom, eine Methylgruppe oder eine Trifluormethylgruppe, $R^2$ ein Wasserstoffatom oder ein Chloratom, A eine Einfachbindung, eine $-CH(R^6)-CH(R^7)$-Gruppe oder eine $-CH(R^6)-CH(R^7)-CH(R^8)$-Gruppe bedeutet, $R^5$ ein Wasserstoffatom oder eine C1–C4-Niederalkylgruppe, einer der Substituenten $R^3$, $R^4$, $R^6$, $R^7$ oder $R^8$ eine Methyl- oder Ethylgruppe darstellt und die anderen Wasserstoffatome bedeuten.

3. Verfahren nach Anspruch 1 zur Herstellung von Phenylalkyloxirancarbonsäuren der allgemeinen Formel I, dadurch gekennzeichnet, dass man Phenylalkyl -2- methylencarbonsäuren der allgemeinen Formel II oxidiert und gegebenenfalls anschliessend die erhaltenen C1–C4-Niederalkylester verseift oder die erhaltenen Säuren in die Salze oder C1–C4-Niederalkylester überführt, wobei $R^1$ und $R^2$ meta- oder para-Substituenten

sind und $R^1$ ein Wasserstoffatom, ein Chloratom oder eine Trifluormethylgruppe, $R^2$ ein Wasserstoffatom, A eine Einfachbindung oder eine $-CH(R^6)-CH(R^7)$-Gruppe bedeutet, $R^5$ ein Wasserstoffatom, eine Methylgruppe oder eine Ethylgruppe bedeutet, einer der Substituenten $R^3$, $R^4$, $R^6$ oder $R^7$ eine Methylgruppe darstellt und die anderen Wasserstoffatome bedeuten.

4. Verfahren nach Anspruch 1 zur Herstellung von Phenylalkyloxirancarbonsäuren der allgemeinen Formel I, dadurch gekennzeichnet, dass man Phenylalkyl -2- methylencarbonsäuren der allgemeinen Formel II oxidiert und gegebenenfalls anschliessend die erhaltenen C1–C4-Niederalkylester verseift oder die erhaltenen Säuren in die Salze oder C1–C4-Niederalkylester überführt, wobei $R^1$ und $R^2$ meta- oder para-Substituenten sind, $R^1$ ein Wasserstoffatom, ein Chloratom oder eine Trifluormethylgruppe, $R^2$ ein Wasserstoffatom, A eine Einfachbindung, eine $-CH(R^6)-CH(R^7)$- oder $-CH(R^6)-CH(R^7)-CH(R^8)-CH(R^9)$-Gruppe, $R^5$ ein Wasserstoffatom oder eine Alkylgruppe mit 1 bis 4 Kohlenstoffatomen bedeutet, einer der Substituenten $R^3$, $R^4$, $R^6$, $R^7$, $R^8$ oder $R^9$ eine Methyl- oder Ethylgruppe und die anderen Wasserstoffatome darstellen.

5. Verfahren nach Anspruch 1 zur Herstellung von Phenylalkyloxirancarbonsäuren der allgemeinen Formel I, dadurch gekennzeichnet, dass man Phenylalkyl -2- methylencarbonsäuren der allgemeinen Formel II oxidiert und gegebenenfalls anschliessend die erhaltenen C1–C4-Niederalkylester verseift oder die erhaltenen Säuren in die Salze oder C1–C4-Niederalkylester überführt, wobei $R^1$ ein para-Substituent ist und ein Wasserstoffatom oder Chloratom darstellt, $R^2$ ein Wasserstoffatom, A eine $-CH(R^6)-CH(R^7)$- oder $-CH(R^6)-CH(R^7)-CH(R^8)-CH(R^9)$-Gruppe, $R^5$ ein Wasserstoffatom oder eine Methyl- oder Ethylgruppe, $R^4$ ein Wasserstoffatom, $R^8$ ein Wasserstoffatom bedeutet, einer der Substituenten $R^3$, $R^6$, $R^7$ oder $R^9$ eine Methyl- oder Ethylgruppe und die anderen Wasserstoffatome darstellen.

**Revendications pour les Etats contractants: BE, CH, DE, FR, GB, IT, LI, LU, NL, SE**

1. Acides phénylalcoyloxiranne-carboxyliques répondant à la formule générale I:

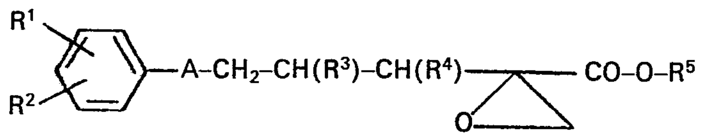

(I),

dans laquelle

$R^1$ représente un atome d'hydrogène, un atome d'halogène, un groupe alcoylique inférieur comportant un à quatre atomes de carbone, un groupe alcoxy inférieur comportant un à quatre atomes de carbone ou un groupe trifluorométhyle,

$R^2$ a l'une des significations de $R^1$,

A représente une liaison simple, un groupe $-CH(R^6)-CH(R^7)$, un groupe $-CH(R^6)-CH(R^7)-CH(R^8)$ ou un groupe $-CH(R^6)-CH(R^7)-CH(R^8)-CH(R^9)$,

$R^5$ représente un atome d'hydrogène ou un groupe alcoylique inférieur comportant un à quatre atomes de carbone, l'un des substituants $R^3$, $R^4$, $R^6$, $R^7$, $R^8$ ou $R^9$ représente un groupe alcoylique inférieur comportant un à quatre atomes de carbone, et les autres un atome d'hydrogène, et les sels de ces acides carboxyliques.

2. Acides phénylalcoyloxiranne-carboxyliques répondant à la formule générale I selon la revendication 1, caractérisés en ce que $R^1$ et $R^2$ sont des substituants méta ou para, et en ce que $R^1$ représente un atome d'hydrogène, un atome de chlore, un groupe méthyle ou un groupe trifluorométhyle, $R^2$ un atome d'hydrogène ou un atome de chlore, A une liaison simple, un groupe, un groupe $-CH(R^6)-CH(R^7)$ ou un groupe $-CH(R^6)-CH(R^7)-CH(R^8)$, $R^5$ un atome d'hydrogène ou un groupe alcoylique inférieur comportant un à quatre atomes de carbone, l'un des substituants $R^3$, $R^4$, $R^6$, $R^7$ ou $R^8$ un groupe méthyle ou éthyle et les autres des atomes d'hydrogène.

3. Acides phénylalcoyloxiranne-carboxyliques répondant à la formule générale I, selon la revendication 1, caractérisés en ce que $R^1$ et $R^2$ sont des substituants méta ou para et en ce que $R^1$ représente un atome d'hydrogène, un atome de chlore ou un groupe trifluorométhyle, $R^2$ un atome d'hydrogène, A une liaison simple ou un groupe $-CH(R^6)-CH(R^7)$, $R^5$ un atome d'hydrogène, un groupe méthyle ou un groupe éthyle, l'un des substituants $R^3$, $R^4$, $R^6$ ou $R^7$, un groupe méthyle et les autres des atomes d'hydrogène, et les sels pharmacologiquement compatibles de ces acides carboxyliques avec des bases minérales et organiques.

4. Acides phénylalcoyloxiranne-carboxyliques répondant à la formule générale I selon la revendication 1, caractérisés en ce que $R^1$ et $R^2$ sont des substituants méta ou para, et en ce que $R^1$ représente un atome d'hydrogène, un atome de chlore ou un groupe trifluorométhyle, $R^2$ un atome d'hydrogène, A une liaison simple, un groupe $-CH(R^6)-CH(R^7)$ ou un groupe $-CH(R^6)-CH(R^7)-CH(R^8)-CH(R^9)$, $R^5$ un atome d'hydrogène ou un groupe alcoyle comportant un à quatre atomes de carbone, l'un des substituants $R^3$, $R^4$, $R^6$, $R^7$, $R^8$ ou $R^9$, un groupe méthyle ou éthyle et les autres des atomes d'hydrogène.

5. Acides phénylalcoyloxiranne-carboxyliques répondant à la formule générale I selon la revendication 1, caractérisés en ce que $R^1$ est un substituant para et représente un atome d'hydrogène ou de chlore, et en ce que $R^2$ représente un atome d'hydrogène, A un groupe $-CH(R^6)-CH(R^7)$ ou

$-CH(R^6)-CH(R^7)-CH(R^8)-CH(R^9)$, $R^5$ un atome d'hydrogène ou un groupe méthyle ou éthyle, $R^4$ un atome d'hydrogène, $R^8$ un atome d'hydrogène, l'un des substituants $R^3$, $R^6$, $R^7$ ou $R^9$ un groupe méthyle ou éthyle et les autres des atomes d'hydrogène, et les sels pharmacologiquement compatibles de ces acides carboxyliques.

6. Procédé de préparation de composés selon la revendication 1, caractérisé en ce que l'on oxyde des acides phénylalcoyl -2- méthylène-carboxyliques répondant à la formule générale II

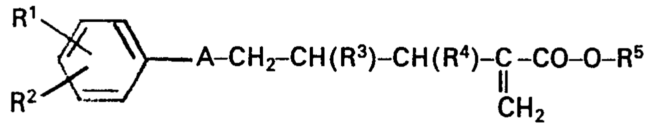
(II),

dans laquelle $R^1$, $R^2$, $R^3$, $R^4$, $R^5$ et A ont la signification indiquée dans la revendication 1, et l'on saponifie éventuellement ensuite les esters alcoyliques inférieurs comportant un à quatre atomes de carbone obtenus, ou l'on transforme les acides obtenus en les sels ou les esters alcoyliques inférieurs comportant un à quatre atomes de carbone.

7. Médicaments, caractérisés en ce qu'ils renferment un ou plusieurs composés selon une ou plusieurs des revendications 1 à 5.

8. Composés selon une ou plusieurs des revendications 1 à 5, caractérisés en ce qu'ils sont appliqués au traitement et à la prophylaxie de maladies qui reposent sur des troubles du métabolisme du glucose et des substances grasses.

**Revendications pour l'Etat contractant: AT**

1. Procédé de préparation d'acides phénylalcoyloxiranne-carboxyliques répondant à la formule générale I

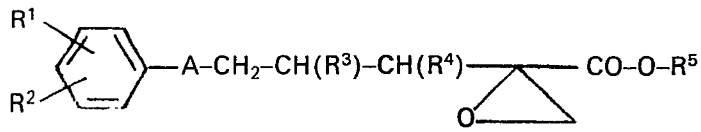
(I),

dans laquelle

$R^1$ représente un atome d'hydrogène, un atome d'halogène, un groupe alcoylique inférieur comportant un à quatre atomes de carbone, un groupe alcoxy inférieur comportant un à quatre atomes de carbone ou un groupe trifluorométhyle,

$R^2$ a l'une des significations de $R^1$,

A représente une liaison simple, un groupe $-CH(R^6)-CH(R^7)$ un groupe $-CH(R^6)-CH(R^7)-CH(R^8)$ ou un groupe $-CH(R^6)-CH(R^7)-CH(R^8)-CH(R^9)$,

$R^5$ un atome d'hydrogène ou un groupe alcoylique inférieur comportant un à quatre atomes de carbone, l'un des substituants $R^3$, $R^4$, $R^6$, $R^7$, $R^8$ ou $R^9$ représente un groupe alcoylique inférieur et les autres un atome d'hydrogène, et des sels de ces acides carboxyliques, caractérisé en ce qu'on oxyde des acides phénylalcoyl -2- méthylène-carboxyliques répondant à la formule générale II

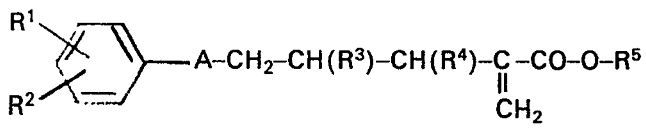
(II),

dans laquelle $R^1$, $R^2$, $R^3$, $R^4$, $R^5$ et A ont la signification indiquée dans la présente revendication 1, et l'on saponifie éventuellement ensuite les esters alcoyliques inférieurs comportant un à quatre atomes de carbone obtenus, ou l'on transforme les acides obtenus en les sels ou les esters alcoyliques inférieurs comportant un à quatre atomes de carbone.

2. Procédé selon la revendication 1 pour préparer des acides phénylalcoyloxiranne-carboxyliques répondant à la formule générale I, caractérisé en ce que l'on oxyde des acides phénylalcoyl -2-méthylène-carboxyliques répondant à la formule générale II et l'on saponifie éventuellement ensuite les esters alcoyliques inférieurs comportant un à quatre atomes de carbone obtenus, ou l'on transforme les acides obtenus en les sels ou les esters alcoyliques inférieurs comportant un à quatre atomes de carbone, $R^1$ et $R^2$ étant des substituants méta ou para, $R^1$ représentant un atome d'hydrogène, un atome de chlore, un groupe méthyle ou un groupe trifluorométhyle, $R^2$ représentant un atome d'hydrogène ou un atome de chlore, tandis que A représente une liaison simple, un groupe $-CH(R^6)-CH(R^7)$ ou un groupe $-CH(R^6)-CH(R^7)-CH(R^8)$, $R^5$ représente un atome d'hydrogène ou un groupe alcoylique inférieur comportant un à quatre atomes de carbone, l'un des substituants $R^3$, $R^4$, $R^6$, $R^7$ ou $R^8$ représente un groupe méthyle ou éthyle, et les autres des atomes d'hydrogène.

3. Procédé selon la revendication 1 pour préparer des acides phénylalcoyloxiranne-carboxyliques répondant à la formule générale I, caractérisé en ce qu'on oxyde des acides phénylalcoyl -2-méthylène-carboxyliques répondant à la formule générale II, et l'on saponifie éventuellement ensuite les esters alcoyliques inférieurs comportant un à quatre atomes de carbone obtenus, ou l'on transforme les acides obtenus en les sels ou les esters alcoyliques inférieurs comportant un à quatre atomes de carbone, $R^1$ et $R^2$ étant des substi-

tuants méta ou para, $R^1$ représentant un atome d'hydrogène, un atome de chlore, ou un groupe trifluorométhyle, $R^2$ un atome d'hydrogène, tandis que A représente une liaison simple ou un groupe $-CH(R^6)-CH(R^7)-$, $R^5$ représente un atome d'hydrogène, un groupe méthyle ou un groupe éthyle, l'un des substituants $R^3$, $R^4$, $R^6$ ou $R^7$ représente un groupe méthyle et les autres des atomes d'hydrogène.

4. Procédé selon la revendication 1 pour préparer des acides phénylalcoyloxiranne-carboxyliques répondant à la formule générale I, caractérisé en ce qu'on oxyde des acides phénylalcoyl -2- méthylène-carboxyliques répondant à la formule générale II et l'on saponifie éventuellement ensuite les esters alcoyliques inférieurs comportant un à quatre atomes de carbone obtenus ou l'on transforme les acides obtenus en les sels ou les esters alcoyliques inférieurs comportant un à quatre atomes de carbone, $R^1$ et $R^2$ étant des substituants méta ou para, $R^1$ représentant un atome d'hydrogène, un atome de chlore ou un groupe trifluorométhyle, $R^2$ représentant un atome d'hydrogène, tandis que A représente une liaison simple, un groupe $-CH(R^6)-CH(R^7)$ ou un groupe $-CH(R^6)-CH(R^7)-CH(R^8)-CH(R^9)$, $R^5$ représente un atome d'hydrogène ou un groupe alcoyle comportant un à quatre atomes de carbone, l'un des substituants $R^3$, $R^4$, $R^6$, $R^7$, $R^8$ ou $R^9$ représente un groupe méthyle ou éthyle et les autres des atomes d'hydrogène.

5. Procédé selon la revendication 1 pour préparer des acides phénylalcoyloxiranne-carboxyliques répondant à la formule générale I, caractérisé en ce qu'on oxyde des acides phénylalcoyl- 2-méthylène-carboxyliques répondant à la formule générale II et l'on saponifie éventuellement ensuite les esters alcoyliques inférieurs comportant un à quatre atomes de carbone obtenus ou bien l'on transforme les acides obtenus en les sels ou les esters alcoyliques inférieurs comportant un à quatre atomes de carbone, $R^1$ étant un substituant para et représentant un atome d'hydrogène ou un atome de chlore, $R^2$ représentant un atome d'hydrogène, A un groupe $-CH(R^6)-CH(R^7)$ ou $-CH(R^6)-CH(R^7)-CH(R^8)-CH(R^9)$, $R^5$ un atome d'hydrogène ou un groupe méthyle ou éthyle, $R^4$ un atome d'hydrogène, $R^8$ un atome d'hydrogène, tandis que l'un des substituants $R^3$, $R^6$, $R^7$ ou $R^9$ représente un groupe méthyle ou éthyle, et les autres des atomes d'hydrogène.

**Claims for the contracting states: BE, CH, DE, FR, GB, IT, LI, LU, NL, SE**

1. Phenylalkyloxiranecarboxylic acids of the general formule I

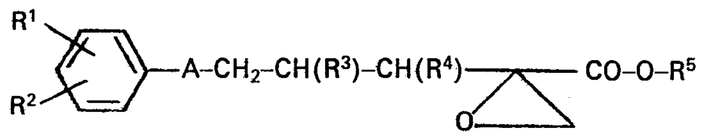

(I),

wherein

$R^1$ denotes a hydrogen atom, a halogen atom, a C1–C4-lower alkyl group, a C1–C4 lower alkoxy group or a trifluoromethyl group,

$R^2$ has one of the meanings of $R^1$,

A denotes a single bond, a $-CH(R^6)-CH(R^7)$-group, a $-CH(R^6)-CH(R^7)-CH(R^8)$- group or a $-CH(R^6)-CH(R^7)-CH(R^8)-CH(R^9)$- group,

$R^5$ denotes a hydrogen atom or a C1–C4 lower alkyl group, one of the substituents $R^3$, $R^4$, $R^6$, $R^7$, $R^8$ or $R^9$ represents a C1–C4-lower alkyl group and the others denote hydrogen atoms, and the salts of the carboxylic acids.

2. Phenylalkyloxiranecarboxylic acids of the general formule I according to claim 1, wherein $R^1$ and $R^2$ are meta- or para-substituents and $R^1$ denotes a hydrogen atom, a chlorine atom, a methyl group or a trifluoromethyl group, $R^2$ denotes a hydrogen atom or a chlorine atom, A denotes a single bond, a $-CH(R^6)-CH(R^7)$-group or a $-CH(R^6)-CH(R^7)-CH(R^8)$-group, $R^5$ denotes a hydrogen atom or a C1–C4-lower alkyl group, one of the substituents $R^3$, $R^4$, $R^6$, $R^7$ or $R^8$ represents a methyl or ethyl group and the others denote hydrogen atoms, and the salts of the carboxylic acids.

3. Phenylalkyloxiranecarboxylic acids of the general formule I according to claim 1, wherein $R^1$ and $R^2$ are meta- or para-substituents and $R^1$ denotes a hydrogen atom, a chlorine atome or a trifluoromethyl group, $R^2$ denotes a hydrogen atom, A denotes a single bond or a $-CH(R^6)-CH(R^7)$-group, $R^5$ denotes a hydrogen atom, a methyl group or an ethyl group, one of the substituents $R^3$, $R^4$, $R^6$ or $R^7$ represents a methyl group and the others denote hydrogen atoms, and the pharmacologically acceptable salts of the carboxylic acids with inorganic and organic bases.

4. Phenylalkyloxiranecarboxylic acids of the general formula I according to claim 1, wherein $R^1$ and $R^2$ are meta- or para-substituents, $R^1$ denotes a hydrogen atom, a chlorine atom or a trifluoromethyl group, $R^2$ denotes a hydrogen atom, A denotes a single bond, a $-CH(R^6)-CH(R^7)$-group or a $-CH(R^6)-CH(R^7)-CH(R^8)-CH(R^9)$-group, $R^5$ denotes a hydrogen atom or an alkyl group with 1 to 4 carbon atoms, one of the substituents $R^3$, $R^4$, $R^6$, $R^7$, $R^8$ or $R^9$ represents a methyl or ethyl group and the others denote hydrogen atomes.

5. Phenylalkyloxiranecarboxylic acids of the general formula I according to claim 1, wherein $R^1$ is a para-substituent and denotes a hydrogen atom or a chlorine atom, $R^2$ denotes a hydrogen atom, A denotes a $-CH(R^6)-CH(R^7)$- or a $-CH(R^6)-CH(R^7)-CH(R^8)-CH(R^9)$-group, $R^5$ denotes a hydrogen atom, a methyl group or an ethyl group, $R^4$ denotes a hydrogen atom, $R^8$ denotes a hydrogen atom, one of the substituents $R^3$, $R^6$, $R^7$ or $R^9$ represents a methyl or ethyl group

and the others denote hydrogen atoms, and the pharmacologically acceptable salts of the carboxylic acids.

6. Process for the preparation of compounds according to Claim 1, characterised in that phenylalkyl -2- methylene-carboxylic acids of the general formula II

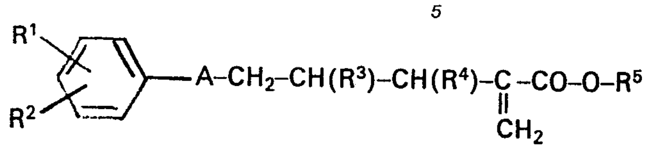

(II),

wherein

$R^1$, $R^2$, $R^3$, $R^4$, $R^5$ and A have the meanings given in Claim 1, are oxidised and, if appropriate, the resulting C1–C4-lower alkyl esters are then hydrolysed, or the resulting acids are then converted into the salts or C1–C4-lower alkyl esters.

7. Medicaments containing one or more compounds according to one or more of claims 1 to 5.

8. Compounds according to one or more of Claims 1 to 5 for use in the treatment and prophylaxis of illnesses based on disorders in glucose metabolism and lipid metabolism.

**Claims for the contracting state: AT**

1. Process for the preparation of phenylalkyl-oxiranecarboxylic acids of the general formula I

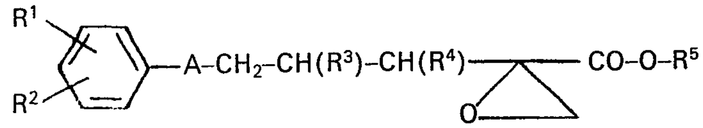

(I),

wherein

$R^1$ denotes a hydrogen atom, a halogen atom, a C1–C4-lower alkyl group, a C1–C4-lower alkoxy group or a trifluoromethyl group,

$R^2$ has one of the meanings of $R^1$,

A denotes a single bond, a $-CH(R^6)-CH(R^7)-$ group, a $-CH(R^6)-CH(R^7)-CH(R^8)-$ group or a $-CH(R^6)-CH(R^7)-CH(R^8)-CH(R^9)-$ group,

$R^5$ denotes a hydrogen atom or a C1–C4-lower alkyl group, one of the substituents $R^3$, $R^4$, $R^6$, $R^7$, $R^8$ or $R^9$ represents a lower alkyl group and the others denote hydrogen atoms, and the salts of the carboxylic acids, characterised in that phenylalkyl -2- methylen-carboxylic acids of the general formula II

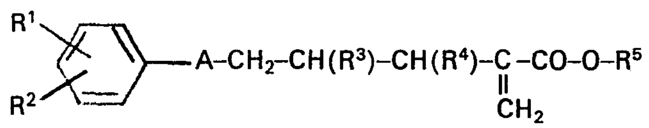

(II),

wherein

$R^1$, $R^2$, $R^3$, $R^4$, $R^5$ and A have the meanings given in Claim 1, are oxidised and, if appropriate, the resulting C1–C4-lower alkyl esters are then hydrolysed, or the resulting acids are then converted into the salts or C1–C4-lower alkyl esters.

2. Process according to claim 1 for the preparation of phenylalkyl-oxiranecarboxylic acids of the general formula I, characterised in that phenylalkyl -2- methylene carboxylic acids of the general formula II are oxidised and, if appropriate, the resulting C1–C4-lower alkyl esters are then hydrolysed, or the resulting acids are then converted into the salts or C1–C4-lower alkyl esters, wherein $R^1$ and $R^2$ meta- or para-substituents and $R^1$ denotes a hydrogen atom, a chlorine atom, a methyl group or a trifluoromethyl group, $R^2$ denotes a hydrogen atom or a chlorine atom, A denotes a single bond, a $-CH(R^6)-CH(R^7)$-group or a $-CH(R^6)-CH(R^7)-CH(R^8)$-group, $R^5$ denotes a hydrogen atom or a C1–C4-lower alkyl group, one of the substituents $R^3$, $R^4$, $R^6$, $R^7$ or $R^8$ represents a methyl or ethyl group and the others denote hydrogen atoms.

3. Process according to claim 1 for the preparation of phenylalkyl-oxiranecarboxylic acids of the general formula I, characterised in that phenylalkyl -2- methylene carboxylic acids of the general formula II are oxidised and, if appropriate, the resulting C1–C4-lower alkyl esters are then hydrolysed, or the resulting acids are then converted into the salts or C1–C4-lower alkyl esters, wherein $R^1$ and $R^2$ are meta- or para-substituents and $R^1$ denotes a hydrogen atom, a chlorine atom or a trifluoromethyl group, $R^2$ denotes a hydrogen atom, A denotes a single bond or a $-CH(R^6)-CH(R^7)$-group, $R^5$ denotes a hydrogen atom, a methyl group or an ethyl group, one of the substituents $R^3$, $R^4$, $R^6$ or $R^7$ represents a methyl group and the others denote hydrogen atoms.

4. Process according to claim 1 for the preparation of phenylalkyl-oxiranecarboxylic acids of the general formula I, characterised in that phenylalkyl -2- methylene carboxylic acids of the

general formula II are oxidised and, if appropriate, the resulting C1–C4-lower alkyl esters are then hydrolysed, or the resulting acids are then converted into the salts or C1–C4-lower alkyl esters, wherein $R^1$ and $R^2$ are meta- or para-substituents, $R^1$ denotes a hydrogen atom, a chlorine atom or a trifluoromethyl group, $R^2$ denotes a hydrogen atom, A denotes a single bond, a $-CH(R^6)-CH(R^7)$-group or a $-CH(R^6)-CH(R^7)-CH(R^8)-CH(R^9)$-group, $R^5$ denotes a hydrogen atom or an alkyl group with 1 to 4 carbon atoms, one of the substituents $R^3$, $R^4$, $R^6$, $R^7$, $R^8$ or $R^9$ represents a methyl or ethyl group and the others denote hydrogen atoms.

5. Process according to claim 1 for the preparation of phenylalkyl-oxiranecarboxylic acids of the general formula I, characterised in that phenylalkyl -2- methylene carboxylic acids of the general formula II are oxidised and, if appropriate, the resulting C1–C4-lower alkyl esters are then hydrolysed, or the resulting acids are then converted into the salts or C1–C4-lower alkyl esters, wherein $R^1$ is a para-substituent and denotes a hydrogen atom or a chlorine atom, $R^2$ denotes a hydrogen atom, A denotes a $-CH(R^6)-CH(R^7)-$ or a $-CH(R^6)-CH(R^7)-CH(R^8)-CH(R^9)$-group, $R^5$ denotes a hydrogen atom, a methyl group or an ethyl group, $R^4$ denotes a hydrogen atom, $R^8$ denotes a hydrogen atom, one of the substituents $R^3$, $R^6$, $R^7$ or $R^9$ represents a methyl or ethyl group and the others denote hydrogen atoms.